# EUROPEAN PATENT SPECIFICATION

(11) **EP 4 591 203 B1**
(45) Date of publication and mention of the grant of the patent: **21.01.2026**
(21) Application number: 24772282.0
(22) Date of filing: 13.09.2024
(51) Int. Cl.: G06F 21/62, G16H 10/60, H04L 9/08

(54) **DISTRIBUTED CLINICAL DATA MANAGEMENT SOLUTION WITH PATIENT-DELEGATED AUTHORIZATION MECHANISM**
VERTEILTE KLINISCHE DATENVERWALTUNGSLÖSUNG MIT PATIENTENABGESCHRÄNKTEM AUTORISIERUNGSMECHANISMUS
SOLUTION DE GESTION DE DONNÉES CLINIQUES DISTRIBUÉES AVEC MÉCANISME D'AUTORISATION DÉPENDANT DU PATIENT

(30) Priority: 19.09.2023 WO PCT/CN2023/119711; 11.10.2023 EP 23202970
(43) Date of publication of application: 30.07.2025
(73) Proprietor: Koninklijke Philips N.V., 5656 AG Eindhoven (NL)
(72) Inventor: ZHANG, Jinsheng, 5656 AG Eindhoven (NL); CHEUNG, Yee Him, 5656 AG Eindhoven (NL)
(74) Representative: Philips Intellectual Property & Standards
(86) International application number: PCT/EP2024/075616
(87) International publication number: WO 2025/061583

(56) References cited:
- GB-A- 2 603 495
- US-A1- 2015 312 759

## Description

### FIELD OF THE INVENTION

This invention relates to the field of medical data management, and in particular to a system that enables a patient to delegate access rights to a plurality of clinical records of the patient in the event that the patient is incapacitated, and a clinician requires access to these clinical records.

### BACKGROUND OF THE INVENTION

One of the biggest problems faced by the healthcare industry is the efficient management and prompt access to a patient's clinical data residing on multiple heterogeneous medical systems. With clinical data scattered over different medical sites, the patient typically does not have a complete view of his/her medical history. Additionally, due to lack of full control over these medical records, the patient is unaware of how the clinical data in the records are utilized. In like manner, it is difficult for clinicians to make correct and informed decisions without a full picture of the patient's medical profile.

Conventionally, a patient's record at each medical site may be uploaded to a 'cloud' server, and accessed as required by clinicians having access to the server. To avoid unauthorized access to the patient's records, security protocols are implemented that allow the patient to control which records at the multiple medical sites may be released to a clinician. The granting of access rights to particular clinicians may be provided in advance of any specific need for such access; however, specific authorization for each access request provides for improved security by avoiding unintentional access authorization. For example, as the patient's clinical records accumulate over time, or as the patient's relationship with the clinician may change, the patient may overlook the need to revoke or modify the a priori authorization.

While the requirement for specific authorization for each access request provides enhanced security for the patient, it introduces the possibility that the patient may be incapacitated at the time that the clinician requires access to the patient's clinical records. Typically, to control the access, the patient must satisfy the implemented security protocol based on a 'secret' known only to the patient, such as a password, decryption key, PIN, etc. To avoid the possibility of failing to provide access to a clinician due to an incapacitation, the patient may share the 'secret' with a delegated person that the patient trusts, and this delegate will grant the access authorization to the clinician by executing the security protocol by 'pretending' to be the patient. However, by providing the secret to the delegate, the delegate may surreptitiously use this secret to access the patient's records at any time, regardless of whether a medical emergency exists, which is not the intent of the patient for sharing the secret. Thus, the level of 'trust' required in the delegate's integrity must be very high.

US 2015/312759 discloses an example of a mobile device which includes a communicator configured to communicate with at least one external device and a controller configured to control decryption of encrypted content that is shared with the at least one external device, in response to determining that the mobile device is located within a proximate spacing of the at least one external device.

### SUMMARY OF THE INVENTION

Since clinical data contain highly sensitive information, a solution is required that enables the patient to control access to the medical records of the patient at the multiple medical sites, while at the same time enabling these records to be accessed when the patient is incapacitated and a medical need exists for providing this emergency access.

Additionally, because consolidating the clinical records of a patient from multiple medical sites onto a common ('cloud') server incurs the risk that a breach of that common server would result in disclosure of all of the patient's clinical records, and incurs significant overhead on the part of each clinical site, a solution is required that does not include the use of a common server.

To better address one or more of these concerns, it would be advantageous to provide a solution that enables each medical site to locally store the patient's clinical data, without storing it on a common server. In order to enable access to all of the clinical records of the patient at the plurality of medical sites that maintain these records, it would also be advantageous to provide a secure means of accessing the clinical records at each of the medical sites by a clinician that is authorized by the patient to access these clinical records at each medical site.

Preferably, each medical site employs a security protocol that uses locally defined access information that enables clinicians from other medical sites to access the patient's clinical data. Upon receipt of a clinician's request for access to the patient's clinical records, the patient selectively provides the locally defined access information for some or all of the requested clinical data to the clinician, and the clinician proceeds to access this clinical data from each identified site.

Typically the access information at each site is specific to each patient, and is related to a pseudo-identifier of the patient (Site-specific Pseudo ID, SPID) that is intended to conceal the patient's actual identify, such as a patient number (or encrypted patient number) in lieu of the patient's name.

To facilitate a linking of the patient's clinical data among each of the medical sites that contain the patient's clinical data, a Patient Data Directory System (PDDS) is provided for use by the patient. As each medical site stores the patient's clinical data, a set of metadata is provided that identifies where the clinical data is stored at the medical site, the access information required to access this clinical data, as well as other data that serves to indicate what information is contained in the clinical data, such as a medical code that identifies the anatomical region related to the clinical data. This metadata is entered into the PDDS by the patient. Access to the PDDS is limited to the patient by a suitable security protocol, such as a challenge-response protocol, an ID-password protocol, etc., except in the case of medical emergency when the patient is incapacitated, as detailed further below.

Preferably, some or all of the metadata related to each clinical record, and particularly the access information, is encrypted by the patient before storage in the PDDS, thereby preventing access to the patient's clinical records in the event of a breach of the PDDS. Any of a variety of techniques may be used to decrypt this information based on a secret known only to the patient, hereinafter termed a 'patient key'. Preferably, the patient key is based on two secrets, wherein a first secret is provided to a patient's delegate, and the second secret is provided to the PDDS. Neither the first nor the second secret is sufficient to determine the patient key, thereby preventing the patient's delegate, the PDDS, or the clinician from accessing the patient's clinical records indiscriminately.

The PDDS is accessible to the clinician for initiating an emergency retrieval of the access information required to access the patient's clinical records when the patient is incapacitated. The PDDS is configured to employ a security protocol that requires the clinician to be 'authenticated' before access to the PDDS is granted.

In an example embodiment, a Data Recovery Device (DRD) is provided to the patient's delegate for recovering, in cooperation with the PDDS, the patient key based on a first secret that is stored at the DRD and a second secret that is stored on the PDDS. The DRD comprises a controller that receives and stores the first secret and executes an emergency access protocol in cooperation with the PDDS in response to a request from a clinician that is authorized to access some or all of the patient's clinical data.

The emergency access protocol is based on a Secure Multiparty Computation (SMC) that enables a determination of the patient key based on an SMC transaction between the DRD (which possesses the first secret), and the PDDS (which possesses the second secret). Of particular note, the SMC transaction does not reveal the first secret to the PDDS, and does not reveal the second secret to the DRD. Upon execution of the SMC transaction, a first output is produced at the DRD, and a second output is produced at the PDDS. A combination of the first and second outputs produces the patient key. The SMC protocol may comprise, for example, a Homomorphic Encryption protocol, a Secret Sharing protocol, or a Garbled Circuit protocol.

In some embodiments, the DRD and the PDDS send the first output and second output, respectively, to the clinician. The clinician combines the first and second outputs to produce the patient key. The PDDS sends the encrypted access information to the clinician, and the clinician decrypts the encrypted access information. The clinician uses the decrypted access information to access the patient's clinical data at the multiple medical sites.

In some embodiments, the DRD sends the first output to the PDDS, and the PDDS combines the first and second outputs to produce the patient key. The PDDS uses the patient key to decrypt the encrypted access information, and sends the decrypted access information to the clinician. In some embodiments, the PDDS executes a proxy re-encryption process to convert the patient-encrypted access information into re-encrypted access information based on a clinician key.

In some embodiments, the PDDS sends the second output and the encrypted access information to the DRD, and the DRD combines the first and second outputs to produce the patient key. The DRD uses the patient key to decrypt the encrypted access information, and sends the decrypted access information to the clinician. In some embodiments, the DRD executes a proxy re-encryption process to convert the patient-encrypted access information into re-encrypted access information based on a clinician key.

Preferably, any device that determines the patient key during the emergency access procedure is configured to delete the patient key from its memory upon completion of the decryption of the patient-encrypted access information.

The first secret may be partitioned and distributed to multiple delegates rather than a single delegate. In this case, upon the clinician's request for emergency access to the patient's clinical data, the single delegate will request the partitions of the secret key from the multiple emergency contacts and reconstruct the first secret. Preferably, an M-out-of-N recovery scheme is used, wherein N is the number of partitions, and M is the minimum number of partitions required to reconstruct the first secret, where M is less than or equal to N. Optionally, any Threshold-based Secure Multiparty Computation scheme may be used.

In some embodiments, the M delegates engage in the SMC with the PDDS.

In some embodiments, the PDDS provides the encrypted access information as an additional input to the SMC, and the SMC provides the decrypted access information to the clinician.

In some embodiments, a Proxy Re-Encryption (PRE) is performed to decrypt the patient-encrypted access information and re-encrypt it to provide a clinician-encryption of the access information that requires a clinician key to decrypt. This re-encryption is performed using a re-encryption key that is based on the patient key and a clinician's private key. This decryption and re-encryption are performed simultaneously, such that a plain-text form of the access information is not produced.

In some embodiments, the key that is recovered from the first and second secrets is a re-encryption key, so as to prevent disclosure of the access information to anyone other than the clinician that possesses the clinician's private key. For ease of reference, the term "emergency-access" key may be used herein to indicate the patient key or the re-encryption key, or any other key that is suitable for removing the patient-encryption from the encrypted metadata during the emergency-access.

Embodiments of this feature include a computer program (or programs) that, when executed by a processing system(s), causes the processing system(s) to perform the above functions of the DRD and PDDS.

### BRIEF DESCRIPTION OF THE DRAWINGS

The invention is explained in further detail, and by way of example, with reference to the accompanying drawings wherein:
FIG. 1 illustrates an overview of the architecture of an example embodiment of a Patient Data Directory System (PDDS).
FIG. 2 illustrates an example flow diagram for providing the patient's clinical data to the clinician, based on the patient's metadata from the PDDS.
FIG. 3 illustrates an example embodiment of a Secure Multiparty Computation (SMC).
FIGs. 4A-4C illustrate example embodiments of a data recovery system that provides emergency decryption of encrypted access information that enables a clinician to access clinical records of a patient at multiple medical sites when the patient is incapacitated.
FIG. 5 illustrates an example flow diagram for re-encrypting the encrypted metadata based on the patient key into encrypted metadata based on the clinician key.
FIGs. 6A-6B illustrate an alternative embodiment that uses re-encryption techniques to produce re-encrypted metadata that can be decrypted by the clinician.
FIGs. 7A-7D illustrate example arrangements of SMC participants in embodiments of this invention.

Throughout the drawings, the same reference numerals indicate similar or corresponding features or functions. The drawings are included for illustrative purposes and are not intended to limit the scope of the invention.

### DETAILED DESCRIPTION

In the following description, for purposes of explanation rather than limitation, specific details are set forth such as the particular architecture, interfaces, techniques, etc., in order to provide a thorough understanding of the concepts of the invention. However, it will be apparent to those skilled in the art that the present invention may be practiced in other embodiments, which depart from these specific details. In like manner, the text of this description is directed to the example embodiments as illustrated in the Figures, and is not intended to limit the claimed invention beyond the limits expressly included in the claims. For purposes of simplicity and clarity, detailed descriptions of well-known devices, circuits, and methods are omitted so as not to obscure the description of the present invention with unnecessary detail.

For ease of understanding, the invention presented herein is presented in the context of the recovery of access data from a patient data directory system (PDDS) that is controlled by the patient to facilitate access by clinicians to clinical data related to the patient when the patient is not able to access the PDDS. One of skill in the art will recognize, however, that the principles of this invention can be applied to other environments and applications.

One of skill in the art will also recognize that the actions of the patient, the clinician, the site, the delegate, etc. described herein will generally be performed using a device configured to perform the actions of the patient, a device configured to perform the actions of the clinician, a device/server configured to perform the actions of the site, a device configured to perform the actions of the delegate', etc. Thus, the terms 'patient' and 'patient device'; 'clinician' and 'clinician device; 'site' and 'site server', 'delegate' and 'delegate device' etc. may be used interchangeably herein. Also, a 'device' may include multiple discrete components, each performing one or more functions of the device.

In like manner, one of skill in the art will recognize that the particular cryptographic methods presented in the example embodiments may be replaced by other cryptographic methods. For example, where feasible, a disclosed symmetric encoding method may be replaced by an asymmetric encoding method to encrypt the material to be protected from disclosure, and vice versa.

For ease of presentation and understanding, details regarding techniques for providing security for the transfer of data, or access to systems and devices, that are not directly related to the principles of this invention are omitted from this description, although one of skill in the art will recognize that such techniques will be included in systems that include this invention to secure such transfers and prevent unauthorized accesses. One of skill in the art will recognize that multiple 'layers' of encryption may be used to encrypt some data elements as the data elements are created and communicated, and in the context of this invention, a 'decryption' removes a particular layer of encryption. That is, any particular decryption will not necessarily provide a plain text output, as detailed further below. Additionally, techniques exist for preventing access to intermediate results in a cryptographic process, and the fact that such results are inaccessible does not affect whether or not such results are produced.

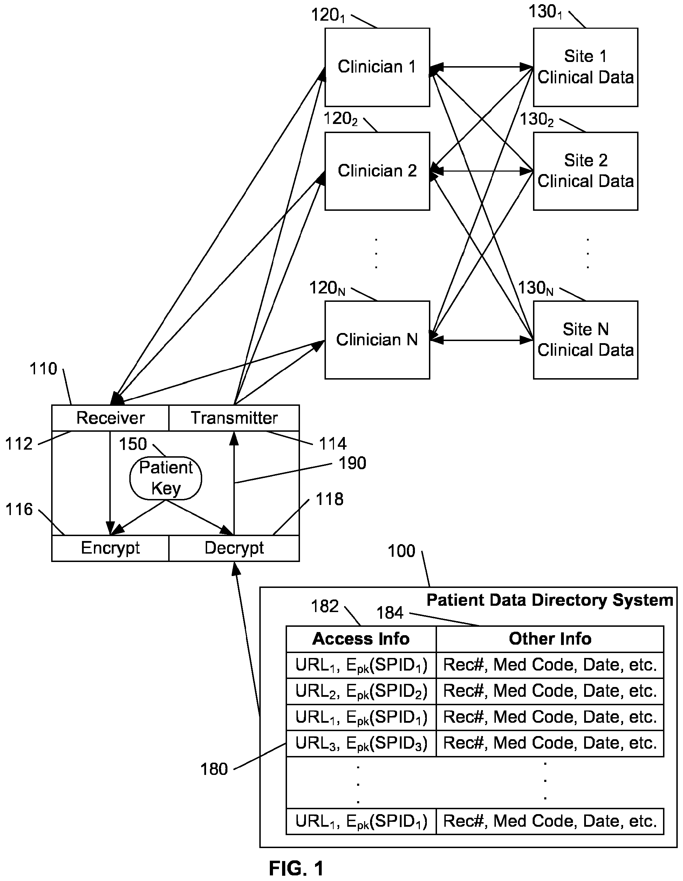

FIG. 1 illustrates an overview of the architecture of an example embodiment of a patient data directory system (PDDS).
WO2024251627A1, provides a description of such an example PDDS. Although this invention is presented using such a PDDS as an example, one of skill in the art will recognize that the principles of this invention may be applied to any set of data that is encrypted by a patient and identifies clinical data of the patient that is required to enable a clinician to access this clinical data. In particular, the set of data may identify clinical data of the patient that is stored at multiple medical sites, and the data includes access information that is required to access each site.

Over time, a patient 110 may visit multiple clinicians 120₁, 120₂, ... 120_{N}, each of these clinicians being associated with a particular medical site. The clinicians store the patient's clinical data 130₁, 130₂, ... 130_{N} at the corresponding medical site. Although FIG. 1 illustrates a one-to-one correspondence between clinicians and sites for ease of understanding, one of skill in the art will recognize that multiple clinicians may be associated with each site. In like manner, each medical site is illustrated as having a single set of clinical data associated with the patient, whereas one of skill in the art would recognize that a medical site may have a variety of different departments, each having clinical records associated with the patient. As used herein, the term 'site' includes 'sub-sites', such as the different departments at a site. Also for ease of understanding, the terms 'clinician' and 'site' may be used interchangeably when referring to actions taken at each site.

To preserve the patient's privacy, each site (or sub-site) identifies the patient using a site-specific identifier of the patient, such as a patient number or other identifier associated with the patient's clinical records, and herein referred to as a Site-specific Pseudo-ID (SPID), which may be encrypted by the medical site. Access to the patient's clinical data at each site requires knowledge of the SPID used at that site. Typically, a patient will identify himself/herself to the clinician, and on the first visit to the medical site, the medical site will assign a pseudo-ID for this site to the patient. Thereafter, the clinician will identify the patient's clinical data using the site-assigned pseudo-ID corresponding to the patient. Note that each site (or sub-site) may assign a different pseudo-ID to the patient, and may employ any of a variety of security techniques to assure that the pseudo-ID does not reveal the actual identity of the patient.

One of skill in the art will recognize use of an SPID is an example form of access information, and that any other form of access information may be used at any or all of the medical sites, such as a password or PIN that is required in order to access the patient's clinical data after the patient is identified to the site.

When the clinician enters the patient's clinical data as a record in the site's storage facility, the clinician, or the site, creates 'metadata' that is associated with the record. In particular, the metadata includes an identification of the record that facilitates access to the record, such as the URL of the particular site, and, optionally, an indication of the location of the record at this URL. Additionally, because the patient's pseudo-ID at the site is required to access the patient's clinical data at the site, the metadata associated with the record also includes access information, such as the patient's pseudo-ID at this particular site.

Additional metadata may include information that characterizes the clinical data that is stored at the record, such as an identification of the anatomic region, a classification of the illness being treated, the date of treatment, and so on. In some embodiments, a set of pre-defined medical 'codes' may be used to classify the content of the record. This additional metadata may be used to 'filter' access to the record. For example, the patient may grant a clinician access to only those records related to a particular anatomic region, or records related to a particular range of dates, and so on.

This metadata for each record of the patient's clinical data at each site is stored in a Patient Data Directory System (PDDS) 100, as detailed further below. As illustrated in FIG. 1, the metadata 180 of each record includes access information 182, such as the site's URL and the patient's SPID. The metadata also includes other metadata 184, as discussed above, that includes any further information required to identify the particular record, as well as information that characterizes the content of the record, such as a medical code, a date, etc.

One of skill in the art will recognize that the particular data structure of the metadata 180 at the PDDS 100 may differ from the linear structure illustrated in FIG. 1. For example, the data structure may be hierarchical, with the site identification (URL and SPID) at a higher level, and each of the specific 'other' metadata 184 associated with each new set of clinical data associated with the patient at this site being 'sub-records' below the site location information. Other structures, including linked-lists and others, may also be used.

Each time a new set of clinical data 130 for the patient 110 is stored at a site, the metadata 180 related to this clinical data is stored at the PDDS 100. In the example of FIG. 1, the metadata 180 for the patient 110 indicates that the patient initially visited Site₁, then visited Site₂, then revisited Site₁, then visited Site₃, etc.

In embodiments of this invention, access to the metadata stored at the PDDS is controlled by the patient. At the patient's first visit to a first clinician, the first clinician 'registers' the patient at the PDDS 100, and in conjunction with the patient 110, provides patient verification information to facilitate secure access to the PDDS 100 by the patient. The PDDS 100 may support multiple patients, and the patient verification information includes an identification of the patient and security information, such as a password or PIN.

As the patient 110 subsequently visits a clinician 120 and a clinical record is created and stored at the corresponding clinical site 120, the clinician 120 communicates the metadata 190 to the patient's receiver 112. To assure security, the patient 110 encrypts 116 some or all of the received metadata 190, such that the decryption of this encrypted metadata 180 requires using a patient key 150 that is preferably known only to the patient. For ease of understanding, the term "encrypted metadata" or "encrypted access information" is used hereinafter to refer to metadata or access information that includes at least one encrypted element, and in particular to metadata or access information that includes at least one encrypted element that is required to access the patient clinical record at the corresponding clinical site 130. The patient submits this encrypted metadata 180 to the PDDS 100 for storage.

For ease of understanding, a single patient key 150 is illustrated. One of skill in the art will recognize that if a public/private key pair is used, the encryption of the SPID will be based on the patient's public key, and the decryption of the SPID will be based on the patient's private key. Such an embodiment will enable, for example, a clinician to encrypt the SPID using the patient's public key before sending the metadata to the patient, thereby avoiding potential disclosure of the access information during the transmission. One of skill in the art will recognize that any of a variety of techniques may be used to securely forward the SPID to the patient. For ease of reference, in the context of this disclosure the term patient key refers to a key or other security item that is required to decrypt the encrypted metadata, and is preferably only known to the patient. In a symmetric encryption, the term 'patient key' refers to the key that is used to encrypt and decrypt the metadata; in an asymmetric encryption (e.g. public/private keys), the 'patient key' refers to the key that is used to decrypt the metadata (e.g. private key).

It is significant to note that the metadata 190 may include multiple layers of encryption, and the decryption using the patient key 150 only removes the encryption produced by the patient. As noted above, the SPID is preferably encrypted by the clinical site using a key associated with the clinical site to produce a site-encrypted SPID that is provided to the patient. This site-encrypted SPID will remain site-encrypted after the removal of the patient-encryption from the encrypted metadata based on the patient key.

As illustrated in FIG. 1, each clinician 120 may access records at multiple sites 130, provided that the patient provides the decrypted metadata of each accessible site to the clinician. Typically, each clinician has read and write access to the site that the clinician is associated with, as indicated by the double-head arrows between each clinician and the associated site. Each clinician typically has read-only access to the sites that the clinician is not associated with, as indicated by the single-head arrow between each clinician and each non-associated site. Preferably, each clinician that is authorized to access information at the clinical sites is provided an authorization token that is used to authenticate the clinician to each clinical site, to gain access and retrieve the clinical data of the patient at each specific medical site identified by the access information.

When a clinician desires access to the patient's records at multiple sites, the clinician submits a request to the patient, and the patient accesses the PDDS to retrieve the metadata related to the request. In some cases, the patient may grant access to the entirety of the records associated with the patient, and will correspondingly provide all of the patient's decrypted metadata to the clinician. The 'request' by the clinician may be implicit, wherein, for example, before a scheduled visit with the clinician, the patient may anticipate that the clinician will request the patient's metadata, and accesses the PDDS to retrieve the metadata before the visit.

In some cases, as noted above, the patient may grant access to only a select set of the patient's records. In such cases, the patient will only provide the decrypted metadata related to selection criteria, such as a span of dates, an anatomical region, etc. The selection criteria may also include exception criteria, wherein the user identifies a class of records that should not be provided to the clinician.

In some embodiments, the PDDS may be configured to store pre-defined selection criteria for some or all of the clinicians that have access to the patient's records and will filter any further data access requests based on these pre-defined selection criteria, unless specifically directed otherwise by the patient.

When the patient 110 agrees to the clinician's request, the patient obtains the desired patient-encrypted metadata 180 from the PDDS 100, decrypts 118 the encrypted metadata 180 using the patient key 150, and transmits 114 the decrypted metadata 190 to the requesting clinician 120. In some embodiments, the patient may encrypt the decrypted metadata using a key associated with the clinician, to preserve security during the transfer process.

Upon receipt of the requested metadata 190 from the patient, the clinician accesses the patient's records at each site identified in the received metadata. The clinician provides the site-specific pseudo-ID (SPID) of the patient, as well as any further access information in each metadata entry to the identified site in the metadata. Given the access information, each site will provide the clinical records of the identified patient to the clinician, but only if the access information is proper and the records at the specified location are associated with the pseudo-ID of the patient.

As noted above, the metadata may have multiple layers of encryption, including encryption based on the clinician's key, encryption based on the clinical site key, etc. One of skill in the art will recognize that at each stage of the process, the respective decryption occurs, even though such decryption is not explicitly mentioned herein.

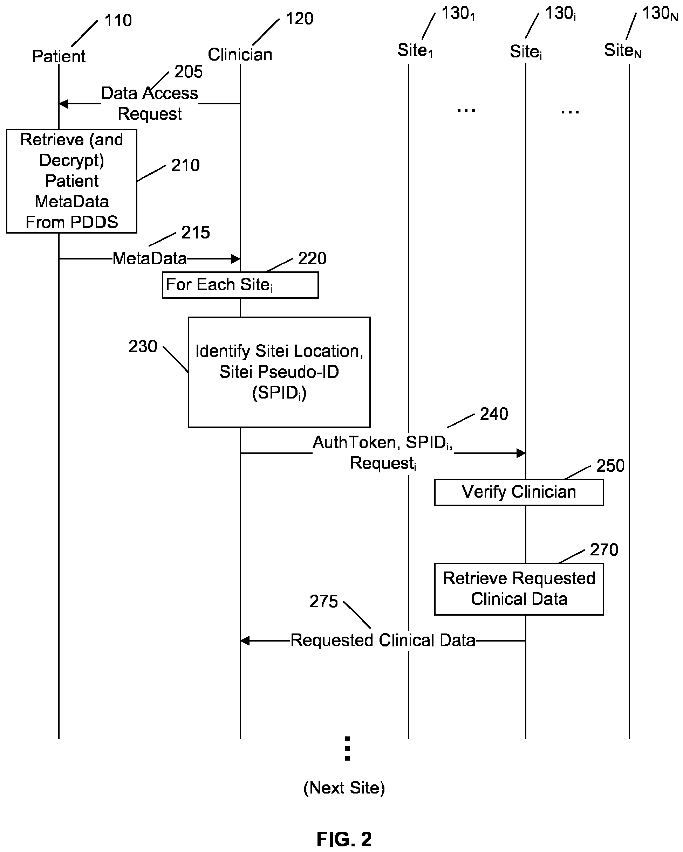

FIG. 2 illustrates an example flow diagram for providing the patient's clinical data to the clinician, based on the patient's metadata from the PDDS.

The clinician 120 initiates the process by sending a data access request 205 to the patient 110. The patient 110 retrieves and decrypts 210 the requested metadata from the PDDS, and sends the decrypted metadata 215 to the clinician 120. In some embodiments (not illustrated), the PDDS may be configured to interact with the patient to effect a re-encryption of the metadata using a key associated with the clinician, and communicates the re-encrypted metadata to the clinician 120 directly, after the patient 110 approves the requested access by the clinician. Typically, the patient's metadata will include multiple entries, each entry corresponding to clinical data stored at a particular site. The clinician iteratively processes 220 the metadata for each site.

At 230, the clinician 120 processes the metadata to identify the Site Locationᵢ (Siteᵢ URL, or SURLᵢ) and the Pseudo-IDᵢ (SPIDᵢ) for the patient 110 at Siteᵢ 130ᵢ. As noted above, the metadata may also include information that facilitates retrieval of the clinical data from the medical site.

The clinician 120 sends 240 his/her authentication information (AuthToken), the SPID_{i,} , and the data request to the identified Siteᵢ. The data request 240 includes the metadata information that facilitates retrieval of the patient's clinical data, as well as other information.

At 250, the Siteᵢ 130ᵢ verifies the clinician 120 based on the AuthToken, and upon successful verification, retrieves 270 the requested clinical data and sends 275 the clinical data to the clinician 120. This process (220 to 275) continues for each site identified in the received metadata 215.

As can be seen, the example PDDS 100 assures that only the patient is able to decrypt the encrypted metadata 180 that is stored at the PDDS 100, and only the patient is able to provide the decrypted metadata 190 that enables a clinician 130 to access the patient's clinical records at the clinical sites 130, in particular to access the patient's clinical records at clinical sites 130 that the clinician is not directly associated with.

While the example PDDS 100 of FIG. 1 provides the patient with excellent security for access to the patient's clinical data, this example PDDS 100 does not enable a clinician 120 to access the patient's clinical data when the patient is incapable of providing the access information required, thereby putting the patient's health at risk by depriving the clinician of clinical data that may be significant in treating the patient.

As noted above, a typical solution to this problem is for the patient to share the patient key and the aforementioned patient verification information for accessing the PDDS with a trusted party, thereby enabling the trusted party to 'impersonate' the patient to the PDDS 100 and provide the decrypted metadata 190 to the clinician. As also noted above, this solution requires a high level of trust in the integrity of the trusted party, and runs the risk of unauthorized disclosure of the patient's clinical data if this level of trust is misplaced, or changes over time.

In a preferred embodiment of this invention, access to and decryption of the information in the PDDS 100 requires that an authenticated clinician identifies that emergency access is required to this information, and that a party that has been delegated by the patient agrees to this access. In this manner, neither the clinician nor the patient's delegate can unilaterally gain access to the patient's clinical data. Also preferably, as contrast with an impersonation solution that would enable the impersonator(s) unlimited access to patient's records at the PDDS 100, the access to the PDDS 100 for such an emergency can be limited to read-only access.

In embodiments of this invention, the patient key 150 is a function of a first secret and a second secret. The patient provides the first secret to patient's delegate, and the second secret to the PDDS. The patient's delegate interacts with the PDDS 100 using a Secure Multiparty Computation (SMC) that enables recovery of the patient key 150 based on the first and second secrets.

As detailed further below, the patient may identify a plurality of delegates, wherein each delegate receives a share of the first secret. For ease of reference, the following description refers to a single delegate, but one of skill in the art will recognize that the 'delegate' may comprise a plurality of delegates. As also detailed below, a re-encryption key may replace the patient key that is recoverable based on the first and second secrets, even though the following description refers to the patient key for ease of reference and understanding.

An advantage of using an SMC to recover the patient key is that the SMC transaction between the delegate (who has the first secret) does not reveal the first secret to the PDDS (which has the second secret), and does not reveal the second secret to the delegate. An SMC also does not require a 'trusted third party' as an intermediary between the delegate and the PDDS.

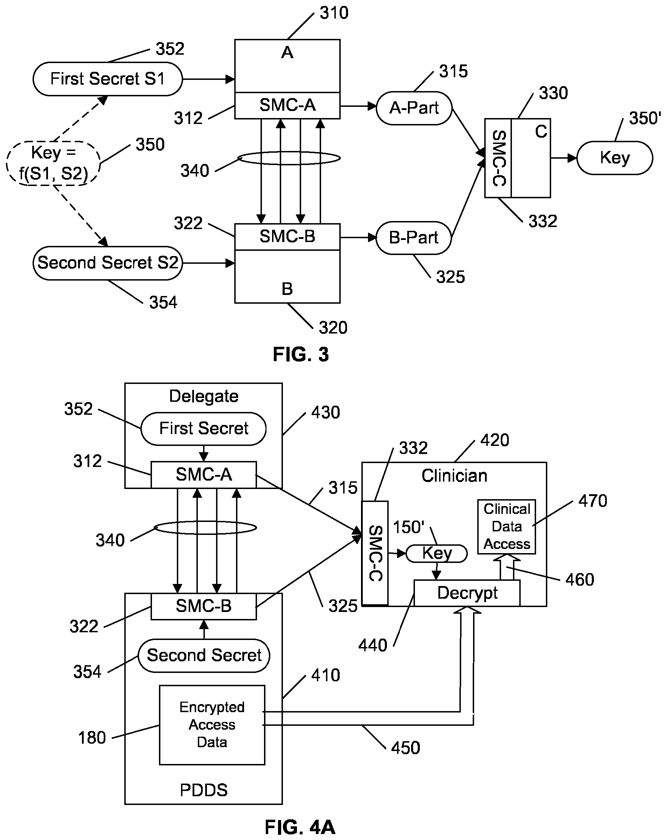

FIG. 3 illustrates an example embodiment of a Secure Multiparty Computation (SMC). As the name implies, an SMC may involve any number of parties, but in the context of this invention, FIG. 3 illustrates a Secure Two-party Computation.

A key 350 is a function of two secrets, a first secret 352, and a second secret 354. Typically, the person P that intends to use the key, such as a patient, selects a first secret S1 and a second secret S2, then applies a function that combines these secrets to form the key 150, such that key=f(S1, S2). The function f may be as simple as an exclusive-OR (XOR) function, or a more complex function, including, for example, a user-defined program written in the Secure Function Definition Language (SFDL) that can be compiled to provide the software components that are provided to the secret-holders to execute the SMC using FairplayMP; similarly JavascriptMPC compiles programs written in Javascript. The function f requires that knowledge of only one secret, S1 or S2, does not reveal the key.

The person P provides the first secret 352 to party A 310, and the second secret 353 to party B 320. Depending on the particular SMC protocol, the person P also communicates the function f() to one or both of the parties A and B. That is, for example, the person P notifies A and/or B that the function f() is an XOR function; or, the nature of the function f() may be publicly known.

To determine the key 350 based on the two secrets 352, 354, parties A and B exchange information, herein termed an SMC transaction 340. The details of the SMC transaction 340 for different SMC protocols is presented further below. For ease of reference, the operations that party A 310 executes during the SMC transaction are referred to as SMC-A 312, and the operations that party B 310 executes are referred to as SMC-B 322.

Although the SMC transaction 340 is illustrated as a two-way exchange between SMC-A and SMC-B, depending upon the particular SMC protocol, the SMC transaction may be a one-way transfer of information between SMC-A and SMC-B.

Upon execution of the SMC transaction 340, SMC-A 312 produces an output, herein termed A-part 315, and SMC-B 322 produces B-part 325. A-part 312 and B-part 325 are sufficient to determine the key 350'.

A-part 315 and B-part 325 are provided to a combiner C 330. Combiner C 330 executes the appropriate operations SMC-C 332 to combine these parts, depending upon the particular SMC protocol used, to recover the key 350' as presented below.

A variety of SMC protocols are available, including Homomorphic Encryption, Secret Sharing, and Garbled Circuit, the details of which are presented below, although one of skill in the art will recognize that other SMC protocols may be used consistent with the features of this invention.

In the following, *a* corresponds to first secret S1 352 in FIG. 3; *b* corresponds to second secret S2 354, and c is equal to f(*a, b*)*,* and corresponds to key 350'.

### HOMOMORPHIC ENCRYPTION

In Homomorphic Encryption (which is asymmetric encryption), the following processes are executed among party A, B, and C:
1. Party A generates a public-private key pair (*pub, priv*)*.*
2. Party A encrypts her input a to be *cipher(a)* with *pub.*
3. Party A sends both *cipher(a)* and *pub* to party B.
4. Party B encrypts his input *b* to be *cipher(b)* with *pub.*
5. Party B evaluates the original function *f* on ciphertext, so that the output *cipher(c)= f(cipher(a), cipher(b))* is a ciphertext of *c*=*f(a,b).*
6. Party A sends the private key *priv* to party C and party B sends *cipher(c)* to party C.
7. Party C decrypts *cipher(c)* with *priv* to obtain *c*.

In this example, steps 1-3 correspond to SMC-A 312 in FIG. 3, with step 3 corresponding to transaction 340; steps 4-5 correspond to SMC-B 322; private key *priv* corresponds to A-Part 315; *cipher(c)* corresponds to B-Part 325, and step 7 corresponds to SMC-C 332.

### SECRET SHARING

In Secret Sharing, the following processes are executed among party A, B, and C:
1. Party A secret shares her input *a* with party B. As a result, party A holds [*a*]_{A} and party B holds [*a*]_{B} where *a* = [*a*]_{A}+[*a*]_{B}. For simplicity, when using [*a*], it means value *a* is in a secret shared format.
2. Party B secret shares his input *b* with party A similarly.
3. Party A and party B evaluate the original function *f* on shared inputs to obtain a secret shared output [*c*] *=f(*[*a*], [*b*])*.*
4. Party A sends [*c*]_{A} to party C, party B sends [*c*]_{B} to party C.
5. Party C computes and obtains *c* = [*c*]_{A} + [*c*]_{B}.

In this example, steps 1 and 3 correspond to SMC-A 312, steps 2 and 3 correspond to SMC-B 322, with steps 1-2 corresponding to transaction 340; [*c*]_{A} corresponds to A-Part 315; [*c*]_{B} corresponds to B-Part 325; and step 5 corresponds to SMC-C 332.

### GARBLED CIRCUIT

Background: Given function *c* = *f*(*a, b),* where *a, b, c* are integers, let *aᵢ* denote the *i*^{th} bit of *a,* so that *a* = *a₁ a₂* ... *a_{N}* (It also applies to *b* and *c*). During circuit generation, each bit is encoded as two random bitstrings called "label." We use *lbl_{i,0}, lbl_{i,1}* to represent the encoding of bit value 0 and 1 for the *i*^{th} bit of *a.* Then, the notation is used to represent all labels for *a,* i.e. {(*lbl_{1,0}, lbl_{1,1}*), *...,* {(*lbl_{N,0}, lbl_{N,1}*)}*.* Thus, the garbled value *a* is denoted as *lbl_{1,a1}, lbl_{2,a2}, ..., lbl_{N,aN}.*

The following processes are executed among party A, B, and C:
1. Party A acts as Garbler to generate garbled circuit *gcirc* corresponding to a function *c*=*f(a,b).* During the process, party A also generates *lbl(a), lbl(b), and lbl(c).*
2. Party A draws *gbl(a)* out of *lbl(a),* then sends *gcirc* and *gbl(a)* to party B acting as Evaluator.
3. Party B obliviously transfers¹ *gbl(b)* out of *lbl(b)* from party A.
4. Party B evaluates the circuit with *gbl(a), gbl(b)* to get garbled output *gbl(c).* At this point, since party A did not send *lbl(b),* party B cannot get the result c in plaintext.
5. Party A sends *lbl(c)* to party C and Party B sends *gbl(c)* to party C.
6. Party C compares *lbl(c)* with *gbl(c)* to obtain the final output *c*.
¹ In cryptography, an oblivious transfer (OT) protocol is a type of protocol in which a sender transfers one of potentially many pieces of information to a receiver, but remains oblivious as to what piece (if any) has been transferred (Wikipedia, Oblivious Transfer).

In this example, steps 1-2 correspond to SMC-A 312, steps 3-4 correspond to SMC-B 322, with steps 2-3 corresponding to transaction 340; *lbl(c)* corresponds to A-Part 315; *gbl(c)* corresponds to B-Part 325; and step 6 corresponds to SMC-C 332.

As noted above, one of skill in the art will recognize that any other variety of SMC protocols may be used in embodiments of this invention, including, for example, Shamir Secret Sharing, Additive Secret Sharing, and others.

FIGs. 4A-4C illustrate example embodiments of a data recovery system that provides emergency decryption of encrypted access information that enables a clinician to access clinical records of a patient at multiple medical sites when the patient is incapacitated. As noted above, the example PDDS is merely used as an example of a patient-controlled data access system that provides for the storage and retrieval of access information that is encrypted by the patient, and is required to access clinical records of the patient at multiple clinical sites. One of skill in the art will recognize that alternative embodiments may be used for such a patient-controlled data access system.

In embodiments, the patient key 150 is based on a first secret 352 and a second secret 354. Typically, when the patient is registered at the PDDS 100, the patient creates the first S1 and second S2 secrets, then uses a cryptography-based function f(S1, S2) to generate the patient key 150. Alternatively, the patient may obtain a patient key 150 and partition it into the first secret S1 and second secret S2.

As illustrated in FIG. 1, the patient uses the patient key (pk) 150 to encrypt the SPIDᵢ associated with the particular clinical siteᵢ to provide Eₚₖ(SPIDᵢ) to the PDDS 100. As noted above, one of skill in the art will recognize that if a public/private key pair is used, the patient may encrypt the SPID using the public key, and decrypt the SPID using the private key; in such an embodiment, the patient key that is to be recovered refers to the patient's private key. Although FIG. 1 illustrates that the SPIDᵢ is encrypted using pk, one or more of the other elements of metadata 180 may also be encrypted. The terms 'encrypted metadata' and 'decrypted metadata' are defined herein as the encryption and decryption of one or more elements of the metadata.

In the embodiment of FIGs. 4A-4C, the patient provides the first secret 352 to a delegate selected by the patient, and provides the second secret 354 to the PDDS 100. Optionally, the patient may provide the second secret to the clinician, and the clinician may provide the second secret to the PDDS upon initiation of the emergency access. The delegate uses a Data Recovery Device (DRD) 430 to interact with the PDDS 410 to recover the patient key 150 based on the first and second secret without revealing the first secret 352 to the PDDS 410 and without revealing the second secret 354 to the DRD 430. The DRD 430 includes components (SMC-A) 312 necessary to execute the SMC transaction 340 with the PDDS 410. The PDDS 410 corresponds to the PDDS 100 of FIG. 1, with the addition of components (SMC-B) 322 necessary to execute the SMC transaction 340 with the DRD 430.

One of skill in the art will recognize that in each embodiment, the functions SMC-A, SMC-B may be interchanged, such that the DRD 430 comprises SMC-B and the PDDS 410 comprises SMC-A.

Preferably, the PDDS is configured to enable an authenticated clinician to execute an emergency access protocol at the PDDS. The clinician preferably contacts the delegate and provides security information to the delegate that enables the delegate to be authenticated to the PDDS and engage in the emergency access protocol. For example, the clinician may generate a private-public key pair and a "token". The clinician signs the random token with the private key and provides the random token to the delegate, and the corresponding signature and the public key to the PDDS. Upon the delegate's submission of the token to the PDDS, the PDDS verifies that the signature corresponds to the token using the clinician's public key, thereby authenticating the delegate.

As noted above, the emergency access protocol is based on a Secure Multiparty Computation (SMC) that enables a determination of the patient key 150' based on an SMC transaction between the PDDS 410 and the delegate's DRD 430. FIGs. 4A-4C illustrate different configurations of the PDDS 410, DRD 430, and clinician device 420 to enable the clinician device to receive the access information required to access the patient's clinical records at a plurality of clinics.

In FIG. 4A, upon execution of the SMC transaction 340 between the DRD 430 and PDDS 410, the SMC-A 312 at the DRD 430 provides a delegate-output 315 (A-Part of FIG. 3) to the clinician device 420, and the PDDS 410 provides an SMC-output 325 (B-Part of FIG. 3) to the clinician device 420. The clinician device 420 includes the components (SMC-C) 332 required to combine the outputs 315, 325 to recover the patient key 150'. The clinician device 420 receives the patient-encrypted metadata 450 from the PDDS 410 and uses the recovered patient key 150' to decrypt 440 the encrypted metadata and produce decrypted metadata 460. The clinician uses this decrypted metadata 460 to access 470 the patient's clinical data from the medical sites indicated in the decrypted metadata 460, using for example, the process illustrated in FIG. 2.

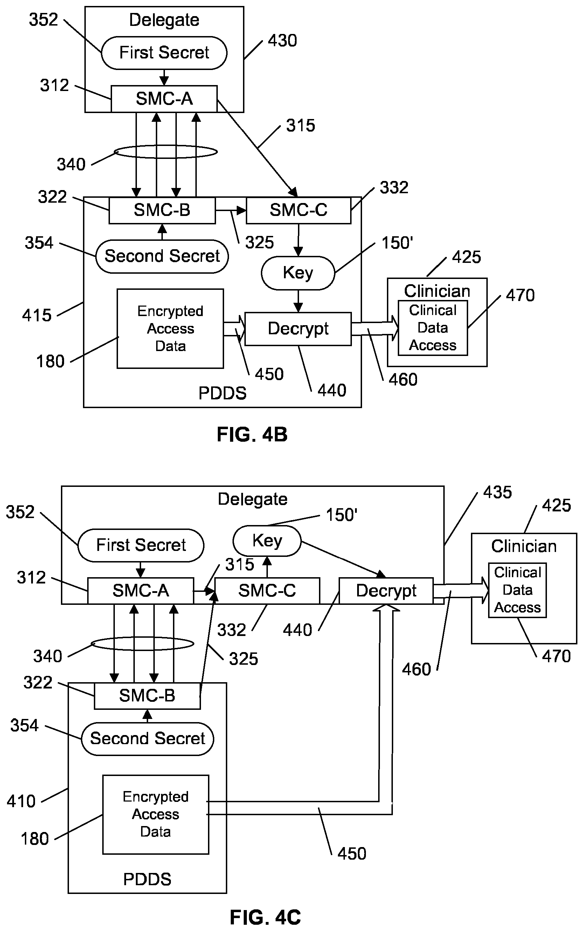

FIG. 4B illustrates an alternative embodiment of the PDDS, wherein the PDDS 415 includes SMC-C 322 components, and is configured to combine the SMC-A delegate-output 315 from the DRD 430, and the SMC-B PDDS-output 325 to recover the patient key 150'. The PDDS 415 uses the key 150' to decrypt 440 the patient-encrypted metadata 450, and sends the decrypted metadata 460 to the clinician device 425. The clinician uses this decrypted metadata 460 to access 470 the patient's clinical data from the medical sites indicated in the decrypted metadata 460.

As noted above, the decrypted metadata 460 may be encrypted using a clinician key to securely transfer the decrypted metadata 460. In embodiments, the PDDS 415 may use Proxy Re-Encryption (PRE) to transform the patient-encrypted metadata 450 into re-encrypted metadata that can be decrypted by the clinician, replacing the decryption at 440 of FIG. 4B with a re-encryption 460 of the metadata that is sent to the clinician. Proxy Re-Encryption allows a first message encrypted by a first user using a first public key to be transformed by a proxy device into a second message that a second user can decrypt, without the proxy device having access to the plain-text (unencrypted) message. That is, the Proxy Re-Encryption process includes the simultaneous decryption based on the patient's key 150' (removal of the patient-encryption) and encryption based on a clinician's key, without producing a plain-text version of the metadata during this decryption-encryption process.

In this case, the patient acquires a public-private key pair (skₚ, pkₚ) and the clinician acquires a public-private key pair (sk_{c}, pk_{c}). During the writing process, the patient encrypts the metadata using the patient's public key (pkₚ) and stores the encrypted metadata E_{PKp}(M) on the PDDS.

During the emergency access protocol, the clinician sends his/her public key pk_{c} to the PDDS, and the PDDS uses a proxy re-encryption scheme to create the re-encryption key rk_{c} based on the recovered patient's private key skₚ 150' and the clinician's public key pk_{c}. The PDDS retrieves the requested patient-encrypted metadata and uses the re-encryption key to transform the patient-encrypted metadata into re-encrypted metadata that can be decrypted by the clinician's private key sk_{c}.

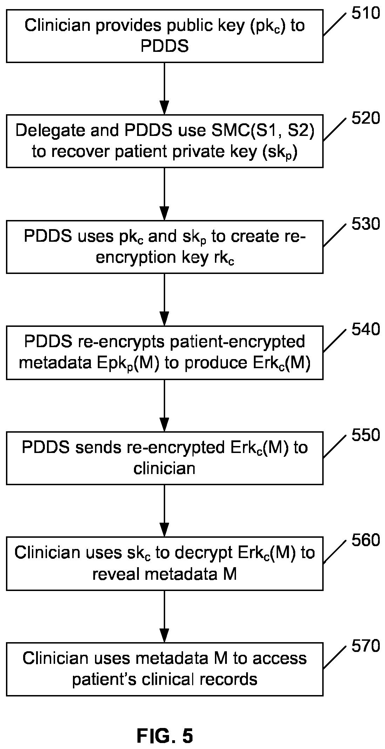

FIG. 5 illustrates an example flow diagram of the re-encryption of patient-encrypted metadata to provide clinician-encrypted metadata.

At 510, the clinician provides the clinician's public key pk_{c} to the PDDS. At 520, the delegate and PDDS engage in an SMC based upon the delegate's first secret S1 and the PDDS's second secret S2 to recover the patient's private key skₚ, as detailed above.

At 530, the PDDS uses Proxy Re-Encryption to create a re-encryption key rk_{c} based on the clinician's public key pk_{c} and the recovered patient's private key skₚ. This re-encryption key rk_{c} enables the PDDS to replace the patient-encryption metadata E_{PKp}(M) with re-encrypted metadata E_{RKc}(M) that can be decrypted using the clinician's private key sk_{c}, at 540, without producing the plain-text metadata M during the Proxy Re-Encryption process.

The PDDS sends this re-encrypted metadata E_{RKc}(M) to the clinician, at 550, and the clinician decrypts this re-encrypted metadata E_{RKc}(M) using the clinician's private key sk_{c} to reveal the metadata M, at 560. As detailed above, this metadata M enables the clinician to access the patient's clinical records at the respective clinical sites, at 570.

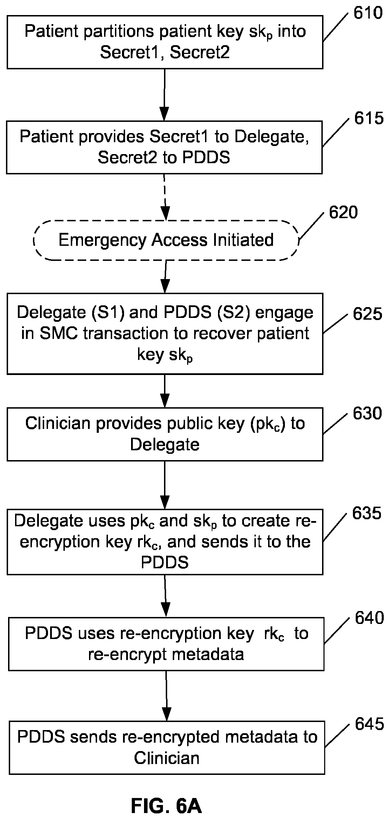

As described further below, FIGs. 6A-6B illustrate alternative embodiments that use Proxy Re-Encryption techniques to produce re-encrypted metadata that can be decrypted by the clinician.

FIG. 4C illustrates an alternative embodiment of the DRD, wherein the DRD 435 includes SMC-C 322 components, and is configured to combine the SMC-A delegate-output 315 and SMC-B PDDS-output 325 from the PDDS 410 to recover the patient key 150'. The DRD 435 receives the patient-encrypted metadata 450 from the PDDS 410, uses the key 150' to decrypt 440 the patient-encrypted metadata 450. The DRD 435 sends the decrypted metadata 460 to the clinician device 425. The clinician uses this decrypted metadata 460 to access 470 the patient's clinical data from the medical sites indicated in the decrypted metadata 460.

The DRD 435 may alternatively be configured to execute a Proxy Re-Encryption to generate the re-encryption key rk_{c} using the recovered patient key skₚ 150' and the clinician's public key pk_{c}. In this case, the clinician provides the clinician's public key pk_{c} to the DRD 435, which replaces the decryption 440 of the patient-encrypted data with the re-encryption of the patient-encrypted metadata 450 from the PDDS to produce the clinician-encrypted metadata 460 that is sent to the clinician. In this manner, the decrypted metadata is not revealed to the delegate.

Optionally (not illustrated), the DRD 435 may send the re-encryption key rk_{c} to the PDDS 410, to enable the PDDS 410 to transform the patient-encrypted metadata 450 into re-encrypted metadata that the clinician can decrypt using the clinician's private key sk_{c} to reveal the decrypted metadata 460.

As stated above, preferably, any device that determines the patient key during the emergency access procedure is configured to avoid disclosure of the patient key to the user of the device, and to delete the patient key from its memory upon completion of the decryption of the patient-encrypted access information. In this manner, the patient key must be recovered based on the first and second secret for each activation of the emergency access protocol.

FIGs. 6A-6B illustrate alternative embodiments that use Proxy Re-Encryption techniques to produce re-encrypted metadata that can be decrypted by the clinician. In these embodiments, the clinician is in possession of a public/private key pair (pk_{c}, sk_{c}). As used herein, the 'clinician' may be a 'clinic' or other medical organization, and all authorized practitioners of that clinic/organization have access to that key pair during an emergency-access event.

In FIG. 6A, steps 610-615 are performed before the emergency access protocol is initiated. As detailed above, the patient partitions the patient key skₚ into two secrets, S1 and S2, at 610. At 620, the patient provides the first secret S1 to the delegate, and the second secret S2 to the PDDS.

When the emergency access protocol is initiated, at 620, the delegate and the PDDS engage in an SMC transaction wherein the delegate provides the first secret S1 and the PDDS provides the second secret S2, and the SMC recovers the patient key skₚ based on S1 and S2, at 625. As detailed above, either the delegate or the PDDS receives the patient key skₚ. In this example, the delegate receives the patient key skₚ.

At 630, the clinician provides the clinician's public key pk_{c} to the delegate (or the PDDS, if the PDDS received the recovered patient key). The delegate uses the public clinician key pk_{c} and the recovered patient key skₚ to create the Proxy Re-Encryption key rk_{c}, at 635.

The delegate provides the re-encryption key rk_{c} to the PDDS, and, at 640, the PDDS uses the re-encryption key rk_{c} to replace the patient-encryption with a clinician-encryption that is based on the clinician's public key pk_{c}, which can be decrypted using the clinician's private key sk_{c}.

At 645, the PDDS provides the re-encrypted metadata to the clinician, to enable the clinician to decrypt the re-encrypted metadata and thereby access the patient's clinical records.

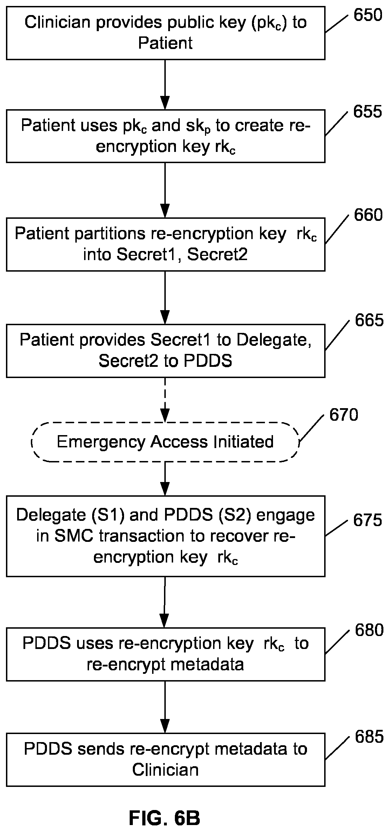

FIG. 6B illustrates an alternative embodiment, wherein the patient creates a re-encryption key rk_{c}, before the emergency-access protocol is initiated, and this re-encryption key is recovered from the secrets S1 and S2, in lieu of the patient key.

Steps 650-665 are performed before the patient is incapacitated, while steps 650-685 are performed during the emergency access process.

In this embodiment, the patient has a public-private key pair (skₚ, pkₚ) and the clinician has a public-private key pair (sk_{c}, pk_{c}). As detailed above, (not illustrated in FIG. 6), the patient encrypts the metadata using the patient's public key pkₚ, and decrypts the metadata using the patient's private key skₚ.

At 650, the clinician provides the clinician's public key pk_{c} to the patient, and the patient creates a re-encryption key rk_{c} based on the patient's private key skₚ and the clinician's public key pk_{c}, at 655. As detailed above, this re-encryption key rk_{c} enables the removal of the patient's encryption and produces a re-encrypted metadata that may be decrypted using the clinician's private key sk_{c}.

At 660, the patient partitions the re-encryption key rk_{c} into a first secret S1, and a second secret S2, and provides the first secret S1 to the delegate, and the second secret to the PDDS, at 665.

When an emergency access is required, at 670, the delegate and the PDDS engage in an SMC based on the first secret S1 and the second secret S2 to recover the re-encryption key rk_{c}, at 675, which is provided to the PDDS. The PDDS uses this re-encryption key rk_{c} to re-encrypt the patient-encrypted metadata into re-encrypted metadata that can be decrypted by the clinician, at 680.

At 685, the PDDS provides this re-encrypted metadata to the clinician, to enable the clinician to decrypt the re-encrypted metadata and thereby access the patient's clinical records.

Note that the above described process provides one re-encryption key rk_{c} and one set of corresponding secrets S1, S2. This embodiment would be suitable, for example, in situations in which the patient identifies one clinician (e.g. the patient's General Practitioner) that is authorized to initiate the emergency access protocol. This embodiment would also be suitable for embodiments wherein, for example, the re-encryption key is based on a public key of the PDDS and the patient's private key. When the delegate and PDDS engage in the SMC to recover the re-encryption key, at 675, the PDDS (or delegate) will use the re-encryption key to remove the patient-encryption and encrypt it based on the PDDS public key. This re-encrypted metadata will be decrypted by the PDDS, or re-encrypted based on the clinician's public key and the PDDS's private key, and this decrypted or re-encrypted metadata will be sent to the clinician. In either event, the patient key is not revealed to the delegate or the PDDS.

In alternative embodiments, a different re-encryption key rk_{c} may be generated for each clinician, then partitioned in such a way to preserve the first secret S1 and generate a corresponding clinician-specific second secret S2_{c}. For example, the first secret S1 may be X-OR'd with the clinician-specific re-encryption key rk_{c} to produce the second secret S2_{c}. The PDDS may be configured to store the plurality of second secrets S2_{c}, indexed by an identifier of the clinician. When the clinician contacts the PDDS to initiate the emergency-access process, the PDDS will use the identifier of the clinician to access the appropriate second secret S2_{c} to use for the SMC(S1, S2_{c}) recovery of the corresponding clinician-specific re-encryption key rk_{c}, and subsequent re-encryption of the patient-encrypted metadata.

Optionally, the second secret S2_{c} may be provided to the clinician when it is created, and the clinician stores this secret S2_{c} in the patient's record. When an emergency access is required, the clinician provides this secret S2_{c} to the PDDS when the clinician initiates the emergency-access protocol, and the PDDS uses this second secret S2_{c} in the SMC(S1, S2_{c}) to recover the clinician-specific re-encryption key rk_{c}.

One of skill in the art will recognize that this embodiment provides enhanced security because even if the emergency access protocol is used to obtain the patient's clinical data, gaining access to this clinical data would also require knowledge of the clinician's private key sk_{c} to decrypt the re-encrypted metadata that is provided by the PDDS using this process.

In some embodiments, after the emergency access, the patient may require the clinician that initiated the emergency access to change his/her key pair and provide the new clinician's public key to the patient so that the patient can create a new re-encryption key and corresponding new second secret. In this manner, the patient may avoid having to change the patient key and consequently re-encrypt all of the metadata in the PDDS based on this new patient key.

As can be seen, the above described interactions require that the particular delegate of the patient be available when the clinician requires emergency access to the patient's clinical data. Alternatively, the patient may share the first secret with a plurality of delegates, but this would require the patient to have sufficient trust in each and every delegate that receives the first secret.

Embodiments of this invention may be enhanced by allowing the patient to share parts of the first secret among a plurality of delegates, wherein a collaboration among the contacts is required to determine the first secret.

Preferably, an M-of-N recovery scheme is used, wherein the first secret is partitioned into N parts and distributed to N delegates. When the emergency access protocol is initiated, the delegate contacts the delegates with a request to receive their corresponding parts of the secret. If the delegate receives at least M of the parts, the delegate executes the recovery scheme to reconstruct the first secret, and proceeds with the SMC process with the PDDS using this reconstructed first secret.

Any of a variety of M-of-N recovery schemes may be used, each with differing levels of security provided, ranging from a highly secure Threshold-based Secure Multiparty Computation (TSMC), such as provided by Advanced MPC^{™} by Sepior, to non-cryptographic recovery schemes.

Preferably, Replicated Secret Sharing (RSS) may be used, as it would provide both almost no computational overhead and threshold recovery properties at the same time. An M-out-of-N RSS scheme allows a secret comprising N shares to be determined upon receipt of at least M shares. If fewer than M shares are received, the secret cannot be determined.

A typical M-out-of-N RSS scheme (N shares where at least M parties together could recover the original secret) can be implemented as follows, where we assume the secret s to be split is an integer and there is a dealer who owns this secret to be shared to N parties, denoted as *P₁*, ... *,P_{N}*:
1. *Split(s):* The dealer computes *s* = *s₁* +... + *s_{N}* and sends [*s*]ᵢ *=* {*si, ... ,s*_{*(n-t*+*i)%n*}} to party *Pᵢ*, for i = *1 to N,* where % represents the modulo function.
*2. Recover (P_{R1}, ..., P_{RT})*: For any subset with T>= M parties, they first elect a representative party. Without loss of generality, suppose *P_{R1}* denotes the representative. All other parties send their [*s*]*_{Ri}* to *P_{R1}.* Then, *P_{R1}* locally computes [*s*] = [*s*]_{R1} υ [*s*]_{R2} υ ... υ [*s*]_{RT}. Lastly, *P_{R1}* adds all elements *sᵢ* in [*s*] to recover the original secret s.

For example, suppose there are 3 parties denoted as *P₁, P₂, P₃,* and an original secret value s is split as *s₁*+*s₂*+*s₃.* [*s*]₁ *=* {*s₁, s₂*} is sent to *P₁*; [*s*]₂ *=* {*s₂, s₃*} is sent to *P₂;* and [*s*]₃ *=* {*s₃, s₁*} is sent to *P₃.* In this example, the combination of any two of the pairs of partitions {*s₁, s₂*}, {*s₂, s₃*}, {*s₃, s₁*} provides parts *s₁, s₂*,and *s₃,* which are sufficient to recover the original secret *s*.

In embodiments of this invention, the patient splits the first secret into N shares (partial secrets) using the above described *Split* step in the M-out-of-N RSS scheme, and distributes the shares to each of N delegates. When the clinician indicates that emergency access is required to the patient's clinical records, the delegate contacts the delegates and requests that they communicate their individual share to him/her. If the delegate obtains at least M shares (including the delegate's share, if the delegate is among the N delegates that received a share), the delegate combines the shares to recover the first secret using the *Recover* step in the M-out-of-N RSS scheme, and continues with the above described emergency access procedure using this determined first secret. A similar process may be used if other M-of-N recovery schemes are used, such as a recovery scheme that creates the first secret based on the partial secrets, rather than splitting the first secret into the partial secrets. Alternatively, each of the M delegates may engage in the SMC with the PDDS to recover the emergency-access key.

Preferably, the DRD is configured to avoid disclosure of the reconstructed first secret to the delegate during this process, and to delete the first secret upon completion of the emergency access protocol. In this manner, the delegate is required to obtain the M parts for each execution of the emergency access protocol.

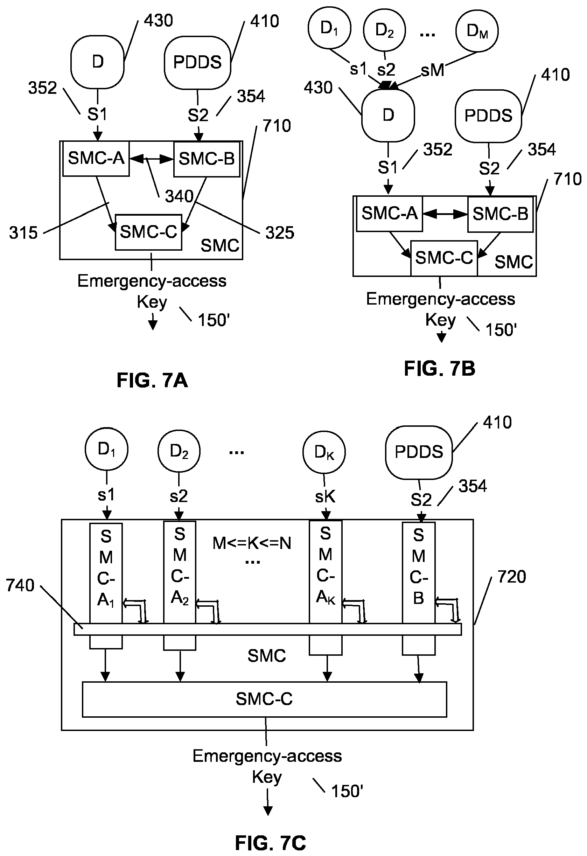

FIGs. 7A-7D illustrate example arrangements of SMC participants in embodiments of this invention. Although the SMCs 710, 720, and 730 in FIGs. 7A-7D are illustrated as a single box, one of skill in the art will recognize that, as detailed above, each SMC comprises components SMC-A, SMC-B, and SMC-C, as illustrated, that may be, and typically are, located on different devices. In some embodiments, these components may be located on a single device, provided that the secret S1 is not disclosed to the party that provides secret S2, and the secret S2 is not disclosed to the party that provides secret S1. In effect, the various elements (delegate device 430, PDDS 410, SMC-A, SMC-B, SMC-C, etc.) disclosed in this invention should be interpreted to be 'logical' elements that each perform their respective logical functions, regardless of where these logical elements are physically situated. One of skill in the art will also recognize that an SMC may comprise other elements, including for example, multiple SMCs arranged in a series or parallel configurations, or a combination of both.

In these examples, the reference numerals of FIG. 3 are used to identify the delegate-output 315, the PDDS-output 325, the interactions 340 between the delegate and the PDDS, the first secret S1 352, and the second secret S2 354.

FIG. 7A illustrates the example presented above wherein the delegate D 430 and the PDDS 410 provide their secrets S1 352, S2 354 to the SMC 710, and the SMC 710 provides the emergency-access key (e.g. patient key or re-encryption key) 150' as the output.

FIG. 7B illustrates the example presented above wherein the delegate D 430 collects partial secrets s1, s2, ... sK, M<=K<=N, from representatives D₁, D₂, ... D_{K} and determines the first secret S1 from these partial secrets, typically using an at least M-out-of-N sharing scheme, as detailed above. As in FIG. 7A, the delegate D 430 provides the first secret S1 and the PDDS 410 provides the second secret S2 to the SMC 710, and the SMC 710 provides the emergency-access key 150' as the output.

Alternatively, the partial secrets s1, s2, ... sK may be provided to an SMC 720 in lieu of the first secret S1, as illustrated in FIG. 7C. In this embodiment, each delegate may have an SMC-A element (e.g. SMC-A₁, SMC-A₂, etc.) of SMC 720. These individual SMC-A elements and the SMC-B element engage in an SMC transaction, as illustrated by communication channel 740, and provide their outputs to SMC-C to recover the emergency-access key 150'. In this manner, as contrast to FIG. 7B, the first secret S1 is not revealed to any of the representatives D₁, D₂, ... D_{M}.

Alternatively, the SMC 720 could directly determine the first secret S1 from the delegates' partial secrets using a conventional M-out-of-N sharing scheme, as the delegate of FIG. 7B, without the delegates engaging in an SMC; but using an SMC to process the shared secrets provides additional security by preventing any of the delegates from learning another delegate's partial secret.

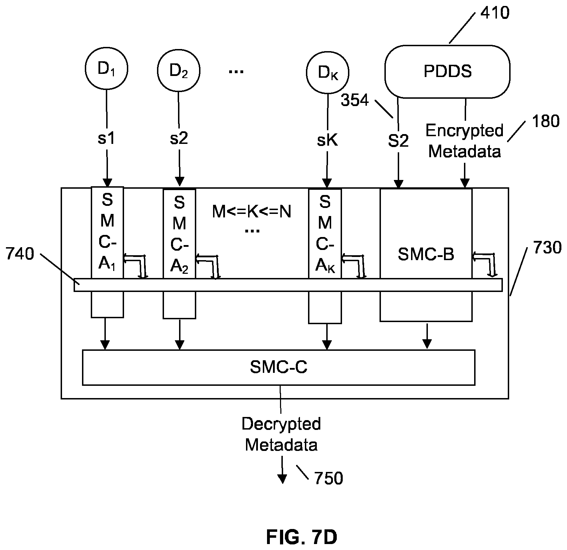

FIG. 7D illustrates another example wherein an SMC 730 receives the partial secrets s1, s2, ... sK from the representatives D₁, D₂, ... D_{K}, M<=K<=N, but in this example, the SMC 730 receives the encrypted metadata 180 as well as the second secret S2 from the PDDS 410. In lieu of outputting the emergency-access key 150', the SMC 730 is configured to perform the decryption of the metadata 180 directly. This decrypted metadata 750 is subsequently provided to the clinician. In this manner, the decrypted metadata 750 is provided to the clinician without the emergency-access key being disclosed to the delegates or the PDDS.

One of skill in the art will recognize that the SMCs 710, 720 illustrated in FIGs. 7A-7C may be similarly modified to also receive the metadata 180 from the PDDS 410 and produce the decrypted metadata 750 for forwarding to the clinician. As noted above, the "decrypted metadata" may in fact be "re-encrypted metadata" that is decrypted using the clinician's private key.

The coordination and execution of the emergency access procedure may take any of a variety of forms. For example, the clinician may communicate the request to execute the emergency access procedure to the delegate and the PDDS. If the first secret is shared among delegates, the delegate will request their shares and recover the first secret. Having been notified by the clinician that emergency access is required, the PDDS will initiate a process to interact with the delegate to generate the respective SMC-A and SMC-B parts. Depending upon the particular embodiment, these parts will be provided to the device containing the SMC-C component. The SMC-C device will determine the emergency-access key, decrypt the patient-encrypted access information, and provide the decrypted access information to the clinician, who will use it to access the patient's clinical records at the medical sites identified in the decrypted access information.

In other embodiments, the identification of the delegate(s) is stored in the PDDS, with their contact information. Upon receipt of the clinician's request for emergency access, the PDDS may contact the delegates and/or delegate(s) to initiate the above described emergency access procedure.

In some embodiments, an independent third party may be used to provide one or more of the above functions. For example, instead of requiring the delegate(s) to interact with the PDDS, the delegate(s) may merely be required to provide the first secret to a DRD operated by a third party. In like manner, the third party may be responsible for contacting the delegate(s) to determine the first secret. Similarly, the recovery of the emergency-access key and the decryption of the patient-encrypted access information may be assigned to a trusted third party.

Other techniques for organizing and managing the execution of the emergency access procedure to recover the emergency-access key in accordance with the principles of this invention will be evident to one of skill in the art.

Preferably, upon recovery, the patient is provided the option of changing the emergency-access key after execution of the emergency access protocol. In an embodiment, to avoid inconveniencing the delegate or emergency contacts, the patient may modify the second secret that is stored in the PDDS and create a new emergency-access key based on this new second secret and the original first secret that is stored at the DRD, or shared among the emergency contacts. The patient may then re-encrypt the encrypted metadata using this new emergency-access key, using for example, a Proxy Re-Encryption process as detailed above. In this manner, the emergency-access key that is derived during execution of the emergency access protocol cannot be re-used, and the subsequent derivation of the new emergency-access key can be performed without requiring the delegate (or emergency contacts) to update the first secret.

Alternatively, to provide additional security, the patient may also change the first secret and distribute the parts of the new first secret to each corresponding emergency contact.

While the invention has been illustrated and described in detail in the drawings and foregoing description, such illustration and description are to be considered illustrative or exemplary and not restrictive; the invention is not limited to the disclosed embodiments.

Of particular note, although the invention is presented as comprising a distinct PDDS 100 that is separate from the patient's device 110 and the medical sites 130, one of skill in the art will recognize that the PDDS 100 may be a centralized service on a cloud server, or within a server at a select medical site 130. In like manner, the PDDS can be a privately managed application that is embodied in the patient device 110. That is, for example, each patient may be provided a PDDS "app" for storing his/her individual metadata records. This PDDS app may be embodied as an application on the patient's personal computer, or, for example, as a "digital wallet" on the patient's mobile device. An advantage of providing an individualized PDDS to each patient is that such an integration will facilitate automation of tasks such as receiving, decrypting, filtering, and forwarding the metadata to the clinician. Such an embodiment also reduces the risks associated with a data breach at a centralized PDDS.

One of skill in the art will recognize that any number of security protocols and systems may be used to augment the security and privacy of embodiments of this invention. For example, some or all of the software and data used may be securely transferred to a Trusted Execution Environment, such as Intel Software Guard Extensions (Intel SGX) which offers hardware-based protection on the specified code execution that isolates specific application code and data in memory, as well as other techniques.

One of skill in the art will also recognize that additional security measures may be applied in embodiments of this invention. For example, even though the invention is presented as being based on two secrets (S1, S2), additional secrets may be required to determine the emergency-access key during an emergency situation. That is, secrets S1, S2 may comprise only a portion of the information required to produce the emergency-access key. Alternatively stated, secrets S1 and S2 are necessary, but may not be sufficient, to produce the emergency-access key. Also alternatively stated, the recovered emergency-access key must be based on at least the secrets S1 and S2, but may also be based on other information. For example, to limit which clinicians are authorized to access the patient data, the patient may split the emergency-access key into three secrets (S1, S2, S3), and provide the secret S3 to each clinician that the patient authorizes to access the patient's clinical data when an emergency access is required.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Also in the claims, the expression "at least one of A, B, and C" means "at least one of A, B, and/or C". A single processor or other unit may fulfill the functions of several items recited in the claims. Similarly, a single function may be performed using multiple processors. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A data recovery system for recovering an emergency-access key (150') of a patient (110) based on a first secret (352) and a second secret (354), comprising:
at least one delegate device (430) that is used by at least one delegate of the patient (110) to provide the first secret (352) to the data recovery system, and
a Patient Data Directory System (PDDS) (100) that provides the second secret (354);
wherein the PDDS (100) is configured to:
store encrypted access information,
wherein the encrypted access information is encrypted by the patient (110),
wherein the encrypted access information comprises access data that enables access by a clinician (120) to clinical data (130) associated with the patient (110) that is stored at a plurality of medical sites;
provide the encrypted access information to the patient (110) upon request from the patient (110), for decryption by the patient (110), and transmission to the clinician (120), thereby enabling the clinician (120) to access the clinical data (130) associated with the patient (110); and
wherein the PDDS and the at least one delegate device (430) are configured to:
execute an emergency access protocol upon request from the clinician (120),
wherein the emergency access protocol is based on a Secure Multiparty Computation (SMC) that enables a determination of the emergency-access key (150') based on an SMC transaction (340) between the PDDS (100) and the at least one delegate device (430),
wherein the SMC transaction (340) does not reveal the first secret (352) to the PDDS, and does not reveal the second secret (354) to the at least one delegate device (430),
wherein the SMC transaction (340) provides at least one delegate-output (315) to the at least one delegate device (430) and a PDDS-output (325) to the PDDS (100),
wherein the emergency access key is recovered based on a combination of the at least one delegate-output (315) and the PDDS-output (325),
wherein the emergency-access key (150') enables decryption of the encrypted access information, thereby enabling the clinician (120) to access the clinical data (130) associated with the patient (110).

2. The data recovery system of claim 1,
wherein the at least one delegate device (430) provides the at least one delegate-output (315) to the clinician (120),
wherein the PDDS (100) provides the PDDS-output (325) to the clinician (120),
wherein the PDDS (100) provides the encrypted access information to the clinician (120),
wherein the clinician combines the at least one delegate-output (315) and the PDDS-output (325) to produce the emergency-access key (150'), and
wherein the clinician (120) decrypts the encrypted access information using the emergency-access key (150').

3. The data recovery system of claim 1,
wherein the at least one delegate device (430) provides the at least one delegate-output (315) to the PDDS,
wherein the PDDS (100) combines the at least one delegate-output (315) and the PDDS-output (325) to produce the emergency-access key (150'), and
wherein the PDDS (100) decrypts the encrypted access information using the emergency-access key (150') to produce decrypted access information (460), and
wherein the PDDS (100) provides the decrypted access information (460) to the clinician (120).

4. The data recovery system of claim 1,
wherein the PDDS (100) provides the PDDS-output (325) to the at least one delegate device (430),
wherein the PDDS (100) provides the encrypted access information to the at least one delegate device (430),
wherein the at least one delegate device (430) combines the at least one delegate-output (315) and the PDDS-output (325) to produce the emergency-access key (150'),
wherein the at least one delegate device (430) decrypts the encrypted access information using the emergency-access key (150'), and
wherein the delegate device (430) provides the decrypted access information (460) to the clinician (120).

5. The data recovery system of claim 1,
wherein the at least one delegate comprises a plurality of delegates,
wherein N partial secrets are distributed to the plurality of delegates,
wherein the first secret (352) can be determined upon receipt of M of the N partial secrets (s1..sK), and cannot be determined based on fewer than M partial secrets (s1..sK),
wherein M is less than or equal to N.

6. The data recovery system of claim 5, wherein one of the plurality of delegates receives at least M partial secrets (s1..sK), determines the first secret (352) based on the at least M partial secrets (s1..sK), and provides the first secret (352) to the SMC.

7. The data recovery system of claim 5, wherein the SMC transaction (340) comprises an SMC computation among the PDDS and at least M delegates.

8. The data recovery system of claim 1, wherein the PDDS (100) provides the encrypted access information to the SMC, and the SMC provides a decryption of the encrypted access information to the clinician (120).

9. The data recovery system of claim 1, wherein the SMC provides the emergency-access key (150') to the at least one delegate device (430).

10. A Patient Data Directory System (PDDS) (100) that stores records of a patient (110) comprising:
a controller that is configured to:
store encrypted access information,
wherein the encrypted access information is encrypted by the patient (110),
wherein the encrypted access information comprises access data that enables access by a clinician (120) to clinical data (130) associated with the patient (110) that is stored at a plurality of medical sites;
provide the encrypted access information to the patient (110) upon request from the patient, for decryption by the patient (110), and transmission to the clinician (120), thereby enabling the clinician (120) to access the clinical data (130) associated with the patient (110); and
execute an emergency access protocol upon request from the clinician (120),
wherein the emergency access protocol is based on a Secure Multiparty Computation (SMC) that enables a determination of an emergency-access key (150') based on an SMC transaction (340) between the PDDS and at least one delegate assigned by the patient (110),
wherein the emergency-access key (150') is a function of a first secret (352) and a second secret (354),
wherein the at least one delegate provides the first secret (352) to the SMC,
wherein the PDDS (100) provides the second secret (354) to the SMC,
wherein the SMC transaction (340) does not reveal the first secret (352) to the PDDS, and does not reveal the second secret (354) to the at least one delegate,
wherein the SMC transaction (340) provides at least one delegate-output (315) to the at least one delegate and a PDDS-output (325) to the PDDS (100),
wherein the emergency-access key (150') is recovered based on a combination of the at least one delegate-output (315) and the PDDS-output (325),
wherein the emergency-access key (150') enables decryption of the encrypted access information, thereby enabling the clinician (120) to access the clinical data (130) associated with the patient (110).

11. The PDDS (100) of claim 10,
wherein the at least one delegate provides the at least one delegate-output (315) to the clinician (120),
wherein the PDDS (100) provides the PDDS-output (325) to the clinician (120),
wherein the PDDS (100) provides the encrypted access information to the clinician (120),
wherein the clinician recovers the emergency-access key (150') based on the at least one delegate-output (315) and the PDDS-output (325), and
wherein the clinician (120) decrypts the encrypted access information using the emergency-access key (150').

12. The PDDS (100) of claim 10,
wherein the at least one delegate provides the at least one delegate-output (315) to the PDDS,
wherein the PDDS (100) recovers the emergency-access key (150') based on the combination of the at least one delegate-output (315) and the PDDS-output (325), and
wherein the PDDS (100) decrypts the encrypted access information using the emergency-access key (150') to produce decrypted access information (460), and
wherein the PDDS (100) provides the decrypted access information (460) to the clinician (120).

13. The PDDS (100) of claim 10,
wherein the PDDS (100) provides the PDDS-output (325) to the at least one delegate,
wherein the PDDS (100) provides the encrypted access information to the at least one delegate,
wherein the at least one delegate recovers the emergency-access key (150') based on the at least one delegate-output (315) and the PDDS-output (325),
wherein the at least one delegate decrypts the encrypted access information using the emergency-access key (150'), and
wherein the at least one delegate provides the decrypted access information (460) to the clinician (120).

14. The PDDS of claim 10, wherein the SMC comprises at least one of: a Homomorphic Encryption protocol, a Secret Sharing protocol, and a Garbled Circuit protocol.

15. A Data Recovery Device (DRD) (430) for recovering access information that enables a clinician (120) to access clinical data (130) associated with a patient (110) that is stored at a plurality of medical sites,
wherein the access information is encrypted by the patient (110) to provide encrypted access information that is stored in a Patient Data Directory System (PDDS) (100),
wherein the DRD is operated by a delegate of the patient (110),
wherein the emergency-access key (150') is a function of a first secret (352) and a second secret (354),
wherein at least a portion of the first secret (352) is stored at the DRD,
wherein the second secret (354) is stored at the PDDS (100),
the DRD comprising:
a controller, wherein the controller is configured to:
receive and store the at least portion of the first secret (352); and
execute an emergency access protocol in cooperation with the PDDS (100) in response to a request from the clinician (120);
wherein the emergency access protocol is based on a Secure Multiparty Computation (SMC) that enables a determination of the emergency-access key (150') based on an SMC transaction (340) between the PDDS (100) and the DRD,
wherein the SMC transaction (340) does not reveal the at least portion of the first secret (352) to the PDDS (100), and does not reveal the second secret (354) to the DRD,
wherein the SMC transaction (340) provides at least one delegate-output (315) to the DRD and a PDDS-output (325) to the PDDS (100),
wherein the emergency-access key (150') is recovered based on a combination of the at least one delegate-output (315) and the PDDS-output (325),
wherein the emergency-access key (150') enables decryption of the encrypted access information, thereby enabling the clinician (120) to access the clinical data (130) associated with the patient (110).

## Patentansprüche

1. Datenwiederherstellungssystem zum Wiederherstellen eines Notfallzugangsschlüssels (150') eines Patienten (110) basierend auf einem ersten Geheimnis (352) und einem zweiten Geheimnis (354), umfassend:
mindestens eine Delegationsvorrichtung (430), die von mindestens einem Beauftragten des Patienten (110) benutzt wird, um das erste Geheimnis (352) an das Datenwiederherstellungssystem bereitzustellen, und
ein Patientendatenverzeichnissystem (PDDS) (100), das das zweite Geheimnis (354) bereitstellt;
wobei das System (100) zu Folgendem konfiguriert ist:
Speichern verschlüsselter Zugangsdaten,
wobei die verschlüsselten Zugangsdaten vom Patienten (110) verschlüsselt werden,
wobei die verschlüsselten Zugangsdaten Zugangsdaten umfassen, die einem Kliniker (120) den Zugang auf klinische Daten (130) ermöglichen, die mit dem Patienten (110) assoziiert sind und in einer Vielzahl medizinischer Standorte gespeichert sind;
Bereitstellen der verschlüsselten Zugangsdaten an den Patienten (110) auf Anfrage des Patienten (110) zur Entschlüsselung durch den Patienten (110) und Übertragung an den Kliniker (120), wodurch der Kliniker (120) auf die mit dem Patienten (110) assoziierten klinischen Daten (130) zugreifen kann; und
wobei das PDDS und die mindestens eine Delegationsvorrichtung (430) zu Folgendem konfiguriert ist:
Ausführen eines Notfallzugangsprotokolls auf Anfrage des Klinikers (120),
wobei das Notfallzugangsprotokoll basierend auf einer sicheren Mehrparteienberechnung (SMC) eine Bestimmung des Notfallzugangsschlüssels (150') basierend auf einer SMC-Transaktion (340) zwischen dem PDDS (100) und der mindestens einen Delegationsvorrichtung (430) ermöglicht,
wobei die SMC-Transaktion (340) das erste Geheimnis (352) gegenüber dem PDDS und das zweite Geheimnis (354) gegenüber mindestens einer Delegationsvorrichtung (430) nicht preisgibt,
wobei die SMC-Transaktion (340) mindestens eine Delegationsausgabe (315) an die mindestens eine Delegationsvorrichtung (430) und eine PDDS-Ausgabe (325) an das PDDS (100) bereitstellt,
wobei der Notfallzugangsschlüssel basierend auf einer Kombination aus der mindestens einen Delegationsausgabe (315) und der PDDS-Ausgabe (325) wiederhergestellt wird,
wobei der Notfallzugangsschlüssel (150') die Entschlüsselung der verschlüsselten Zugangsinformationen ermöglicht, wodurch der Kliniker (120) auf die mit dem Patienten (110) assoziierten klinischen Daten (130) zugreifen kann.

2. Datenwiederherstellungssystem nach Anspruch 1,
wobei die mindestens eine Delegationsvorrichtung (430) die mindestens eine Delegationsausgabe (315) an den Kliniker (120) bereitstellt,
wobei das PDDS (100) die PDDS-Ausgabe (325) an den Kliniker (120) bereitstellt,
wobei das PDDS (100) die verschlüsselten Zugangsdaten an den Kliniker (120) bereitstellt,
wobei der Kliniker die mindestens eine Delegationsausgabe (315) und die PDDS-Ausgabe (325) kombiniert, um den Notfallzugangsschlüssel (150') herzustellen, und
wobei der Kliniker (120) die verschlüsselten Zugangsdaten unter Verwendung des Notfallzugangsschlüssels (150') entschlüsselt.

3. Datenwiederherstellungssystem nach Anspruch 1,
wobei die mindestens eine Delegationsvorrichtung (430) die mindestens eine Delegationsausgabe (315) an das PDDS bereitstellt,
wobei das PDDS (100) die mindestens eine Delegationsausgabe (315) und die PDDS-Ausgabe (325) kombiniert, um den Notfallzugangsschlüssel (150') herzustellen, und
wobei das PDDS (100) die verschlüsselten Zugangsinformationen unter Verwendung des Notfallzugangsschlüssels (150') entschlüsselt, um entschlüsselte Zugangsinformationen (460) herzustellen, und
wobei das PDDS (100) die entschlüsselten Zugangsinformationen (460) an den Kliniker (120) bereitstellt.

4. Datenwiederherstellungssystem nach Anspruch 1,
wobei das PDDS (100) die PDDS-Ausgabe (325) an die mindestens eine Delegationsvorrichtung (430) bereitstellt,
wobei das PDDS (100) die verschlüsselten Zugangsinformationen an die mindestens eine Delegationsvorrichtung (430) bereitstellt,
wobei die mindestens eine Delegationsvorrichtung (430) die mindestens eine Delegationsausgabe (315) und die PDDS-Ausgabe (325) kombiniert, um den Notfallzugangsschlüssel (150') herzustellen,
wobei die mindestens eine Delegationsvorrichtung (430) die verschlüsselten Zugangsinformationen unter Verwendung des Notfallzugangsschlüssels (150') entschlüsselt, und
wobei die Delegationsvorrichtung (430) die entschlüsselten Zugangsinformationen (460) an den Kliniker (120) bereitstellt.

5. Datenwiederherstellungssystem nach Anspruch 1,
wobei der mindestens eine Delegierte eine Vielzahl von Delegierten umfasst,
wobei N Teilgeheimnisse an die Vielzahl von Delegierten verteilt werden,
wobei das erste Geheimnis (352) nach Erhalt von M der N Teilgeheimnisse (s1...sK) bestimmt werden kann und nicht basierend auf weniger als M Teilgeheimnissen (s1...sK) bestimmt werden kann,
wobei M unterhalb oder gleich N ist.

6. Datenwiederherstellungssystem nach Anspruch 5, wobei einer der Anzahl der Beauftragten mindestens M Teilgeheimnisse (s1...sK) empfängt, das erste Geheimnis (352) basierend auf den mindestens M Teilgeheimnissen (s1...sK) bestimmt und das erste Geheimnis (352) an den SMC bereitstellt.

7. Datenwiederherstellungssystem nach Anspruch 5, wobei die SMC-Transaktion (340) eine SMC-Berechnung zwischen dem PDDS und mindestens M Delegierten umfasst.

8. Datenwiederherstellungssystem nach Anspruch 1, wobei das PDDS (100) die verschlüsselten Zugangsinformationen an die SMC bereitstellt und die SMC eine Entschlüsselung der verschlüsselten Zugangsinformationen an den Kliniker (120) bereitstellt.

9. Datenwiederherstellungssystem nach Anspruch 1, wobei die SMC den Notfallzugangsschlüssel (150') an die mindestens eine Delegationsvorrichtung (430) bereitstellt.

10. Patientendatenverzeichnissystem (PDDS) (100), das Aufzeichnungen eines Patienten (110) speichert, umfassend:
eine Steuereinheit, die zu Folgendem konfiguriert ist:
Speichern verschlüsselter Zugangsdaten,
wobei die verschlüsselten Zugangsdaten vom Patienten (110) verschlüsselt werden,
wobei die verschlüsselten Zugangsdaten Zugangsdaten umfassen, die einem Kliniker (120) den Zugang auf klinische Daten (130) ermöglichen, die mit dem Patienten (110) assoziiert sind und in einer Vielzahl medizinischer Standorte gespeichert sind;
Bereitstellen der verschlüsselten Zugangsdaten an den Patienten (110) auf Anfrage des Patienten zur Entschlüsselung durch den Patienten (110) und Übertragung an den Kliniker (120), wodurch der Kliniker (120) auf die mit dem Patienten (110) assoziierten klinischen Daten (130) zugreifen kann; und
Ausführen eines Notfallzugangsprotokolls auf Anfrage des Klinikers (120),
wobei das Notfallzugangsprotokoll basierend auf einer sicheren Mehrparteienberechnung (SMC) einen Notfallzugangsschlüssel (150') basierend auf einer SMC-Transaktion (340) zwischen dem PDDS und mindestens einem vom Patienten (110) zugewiesenen Delegierten bestimmt,
wobei der Notfallzugangsschlüssel (150') eine Funktion eines ersten Geheimnisses (352) und eines zweiten Geheimnisses (354) ist,
wobei der mindestens eine Delegierte das erste Geheimnis (352) an den SMC bereitstellt,
wobei das PDDS (100) das zweite Geheimnis (354) an den SMC bereitstellt,
wobei die SMC-Transaktion (340) das erste Geheimnis (352) nicht an das PDDS und das zweite Geheimnis (354) nicht an den mindestens einen Delegierten preisgibt,
wobei die SMC-Transaktion (340) dem mindestens einen Delegierten mindestens eine Delegationsausgabe (315) und dem PDDS (100) eine PDDS-Ausgabe (325) bereitstellt,
wobei der Notfallzugangsschlüssel (150') basierend auf einer Kombination aus der mindestens einen Delegationsausgabe (315) und der PDDS-Ausgabe (325) wiederhergestellt wird,
wobei der Notfallzugangsschlüssel (150') die Entschlüsselung der verschlüsselten Zugangsinformationen ermöglicht, wodurch der Kliniker (120) auf die mit dem Patienten (110) assoziierten klinischen Daten (130) zugreifen kann.

11. PDDS (100) nach Anspruch 10,
wobei der mindestens eine Delegierte die mindestens eine Delegationsausgabe (315) an den Kliniker (120) bereitstellt,
wobei das PDDS (100) die PDDS-Ausgabe (325) an den Kliniker (120) bereitstellt,
wobei das PDDS (100) die verschlüsselten Zugangsdaten an den Kliniker (120) bereitstellt,
wobei der Kliniker den Notfallzugangsschlüssel (150') basierend auf der mindestens einen Delegationsausgabe (315) und der PDDS-Ausgabe (325) wiederherstellt, und
wobei der Kliniker (120) die verschlüsselten Zugangsdaten unter Verwendung des Notfallzugangsschlüssels (150') entschlüsselt.

12. PDDS (100) nach Anspruch 10,
wobei der mindestens eine Delegierte die mindestens eine Delegationsausgabe (315) an das PDDS bereitstellt,
wobei das PDDS (100) den Notfallzugangsschlüssel (150') basierend auf der Kombination aus der mindestens einen Delegationsausgabe (315) und der PDDS-Ausgabe (325) wiederherstellt, und
wobei das PDDS (100) die verschlüsselten Zugangsinformationen unter Verwendung des Notfallzugangsschlüssels (150') entschlüsselt, um entschlüsselte Zugangsinformationen (460) herzustellen, und
wobei das PDDS (100) die entschlüsselten Zugangsinformationen (460) an den Kliniker (120) bereitstellt.

13. PDDS (100) nach Anspruch 10,
wobei das PDDS (100) die PDDS-Ausgabe (325) an den mindestens einen Delegierten bereitstellt,
wobei das PDDS (100) die verschlüsselten Zugangsinformationen an den mindestens einen Delegierten bereitstellt,
wobei der mindestens eine Delegierte den Notfallzugangsschlüssel (150') basierend auf der mindestens einen Delegationsausgabe (315) und der PDDS-Ausgabe (325) wiederherstellt,
wobei der mindestens eine Delegierte die verschlüsselten Zugangsinformationen unter Verwendung des Notfallzugangsschlüssels (150') entschlüsselt, und
wobei der mindestens eine Delegierte die entschlüsselten Zugangsinformationen (460) an den Kliniker (120) bereitstellt.

14. PDDS nach Anspruch 10, wobei die SMC mindestens eines der folgenden Elemente umfasst: ein homomorphes Verschlüsselungsprotokoll, ein Geheimnisteilungsprotokoll und ein Garbled-Circuit-Protokoll.

15. Datenwiederherstellungsvorrichtung (DRD) (430) zum Wiederherstellen von Zugangsinformationen, die es einem Kliniker (120) ermöglicht, auf klinische Daten (130) zuzugreifen, die mit einem Patienten (110) in Verbindung stehen und an mehreren medizinischen Standorten gespeichert sind,
wobei die Zugangsinformationen vom Patienten (110) verschlüsselt werden, um verschlüsselte Zugangsinformationen bereitzustellen, die in einem Patientendatenverzeichnissystem (PDDS) (100) gespeichert werden,
wobei die DRD von einem Delegierten des Patienten (110) betrieben wird,
wobei der Notfallzugangsschlüssel (150') eine Funktion eines ersten Geheimnisses (352) und eines zweiten Geheimnisses (354) ist,
wobei mindestens ein Abschnitt des ersten Geheimnisses (352) in der DRD gespeichert ist,
wobei das zweite Geheimnis (354) im PDDS (100) gespeichert ist,
wobei die DRD Folgendes umfasst:
eine Steuereinheit, wobei die Steuereinheit zu Folgendem konfiguriert ist:
Empfangen und Speichern des mindestens einen Abschnitts des ersten Geheimnisses (352); und
Ausführung eines Notfallzugangsprotokolls in Zusammenarbeit mit dem PDDS (100) als Reaktion auf eine Anfrage des Klinikers (120);
wobei das Notfallzugangsprotokoll auf einer sicheren Mehrparteienberechnung (SMC) basiert, die eine Bestimmung des Notfallzugangsschlüssels (150') basierend auf einer SMC-Transaktion (340) zwischen dem PDDS (100) und der DRD ermöglicht,
wobei die SMC-Transaktion (340) den mindestens einen Abschnitt des ersten Geheimnisses (352) nicht an das PDDS (100) und das zweite Geheimnis (354) nicht an die DRD preisgibt,
wobei die SMC-Transaktion (340) mindestens eine Delegationsausgabe (315) an die DRD und eine PDDS-Ausgabe (325) an die PDDS (100) bereitstellt,
wobei der Notfallzugangsschlüssel (150') basierend auf einer Kombination aus der mindestens einen Delegationsausgabe (315) und der PDDS-Ausgabe (325) wiederhergestellt wird,
wobei der Notfallzugangsschlüssel (150') die Entschlüsselung der verschlüsselten Zugangsinformationen ermöglicht, wodurch der Kliniker (120) auf die mit dem Patienten (110) assoziierten klinischen Daten (130) zugreifen kann.

## Revendications

1. Système de récupération de données permettant de récupérer une clé d'accès d'urgence (150') d'un patient (110) sur la base d'un premier secret (352) et d'un second secret (354) comprenant :
au moins un dispositif délégué (430) qui est utilisé par au moins un délégué du patient (110) pour fournir le premier secret (352) au système de récupération de données, et
un système de répertoire de données de patients (PDDS) (100) qui fournit le second secret (354) ;
dans lequel le PDDS (100) est configuré pour :
stocker des informations d'accès cryptées,
dans lequel les informations d'accès cryptées sont cryptées par le patient (110),
dans lequel les informations d'accès cryptées comprennent des données d'accès qui permettent à un clinicien (120) d'accéder à des données cliniques (130) associées au patient (110) qui sont stockées sur une pluralité de sites médicaux ;
fournir les informations d'accès chiffrées au patient (110) sur demande de ce dernier, pour déchiffrement par le patient (110) et transmission au clinicien (120), permettant ainsi à ce dernier (120) d'accéder aux données cliniques (130) associées au patient (110) ; et
dans lequel le PDDS et le au moins un dispositif délégué (430) sont configurés pour :
exécuter un protocole d'accès d'urgence à la demande du clinicien (120),
dans lequel le protocole d'accès d'urgence est basé sur un calcul multipartite sécurisé (SMC) qui permet une détermination de la clé d'accès d'urgence (150') sur la base d'une transaction SMC (340) entre le PDDS (100) et le au moins un dispositif délégué (430),
dans lequel la transaction SMC (340) ne révèle pas le premier secret (352) au PDDS, et ne révèle pas le second secret (354) à au moins un dispositif délégué (430),
dans lequel la transaction SMC (340) fournit au moins une sortie de délégué (315) à le au moins un dispositif délégué (430) et une sortie de PDDS (325) au PDDS (100),
dans lequel la clé d'accès d'urgence est récupérée sur la base d'une combinaison de la au moins une sortie de délégué (315) et de la sortie de PDDS (325),
dans lequel la clé d'accès d'urgence (150') permet le décryptage des informations d'accès cryptées, permettant ainsi au clinicien (120) d'accéder aux données cliniques (130) associées au patient (110).

2. Système de récupération de données selon la revendication 1,
dans lequel le au moins un dispositif délégué (430) fournit la au moins une sortie de délégué (315) au clinicien (120),
dans lequel le PDDS (100) fournit la sortie de PDDS (325) au clinicien (120),
dans lequel le PDDS (100) fournit les informations d'accès cryptées au clinicien (120),
dans lequel le clinicien combine la au moins une sortie de délégué (315) et la sortie de PDDS (325) pour produire la clé d'accès d'urgence (150') et
dans lequel le clinicien (120) décrypte les informations d'accès cryptées à l'aide de la clé d'accès d'urgence (150').

3. Système de récupération de données selon la revendication 1,
dans lequel le au moins un dispositif délégué (430) fournit la au moins une sortie de délégué (315) au PDDS,
dans lequel le PDDS (100) combine la au moins une sortie de délégué (315) et la sortie de PDDS (325) pour produire la clé d'accès d'urgence (150') et
dans lequel le PDDS (100) décrypte les informations d'accès cryptées à l'aide de la clé d'accès d'urgence (150') pour produire des informations d'accès décryptées (460), et
dans lequel le PDDS (100) fournit les informations d'accès décryptées (460) au clinicien (120).

4. Système de récupération de données selon la revendication 1,
dans lequel le PDDS (100) fournit la sortie de PDDS (325) à le au moins un dispositif délégué (430),
dans lequel le PDDS (100) fournit les informations d'accès cryptées à le au moins un dispositif délégué (430),
dans lequel le au moins un dispositif délégué (430) combine la au moins une sortie de délégué (315) et la sortie de PDDS (325) pour produire la clé d'accès d'urgence (150'),
dans lequel le au moins un dispositif délégué (430) décrypte les informations d'accès cryptées à l'aide de la clé d'accès d'urgence (150'), et
dans lequel le dispositif délégué (430) fournit les informations d'accès décryptées (460) au clinicien (120).

5. Système de récupération de données selon la revendication 1,
dans lequel le au moins un délégué comprend une pluralité de délégués,
dans lequel N secrets partiels sont distribués à la pluralité de délégués,
dans lequel le premier secret (352) peut être déterminé à la réception de M des N secrets partiels (s1...sK), et ne peut pas être déterminé sur la base de moins de M secrets partiels (s1...sK),
dans lequel M est inférieur ou égal à N.

6. Système de récupération de données selon la revendication 5, dans lequel l'un de la pluralité de délégués reçoit au moins M secrets partiels (s1...sK), détermine le premier secret (352) sur la base des au moins M secrets partiels (s1...sK), et fournit le premier secret (352) au SMC.

7. Système de récupération de données selon la revendication 5, dans lequel la transaction SMC (340) comprend un calcul SMC entre le PDDS et au moins M délégués.

8. Système de récupération de données selon la revendication 1, dans lequel le PDDS (100) fournit les informations d'accès cryptées au SMC, et le SMC fournit un décryptage des informations d'accès cryptées au clinicien (120).

9. Système de récupération de données selon la revendication 1, dans lequel le SMC fournit la clé d'accès d'urgence (150') à le au moins un dispositif délégué (430).

10. Système de répertoire de données de patients (PDDS) (100) qui stocke les dossiers d'un patient (110) comprenant :
un dispositif de commande qui est configuré pour :
stocker des informations d'accès cryptées,
dans lequel les informations d'accès cryptées sont cryptées par le patient (110),
dans lequel les informations d'accès cryptées comprennent des données d'accès qui permettent à un clinicien (120) d'accéder à des données cliniques (130) associées au patient (110) qui sont stockées sur une pluralité de sites médicaux ;
fournir les informations d'accès cryptées au patient (110) sur demande du patient, pour décryptage par le patient (110) et transmission au clinicien (120), permettant ainsi au clinicien (120) d'accéder aux données cliniques (130) associées au patient (110) ; et
exécuter un protocole d'accès d'urgence à la demande du clinicien (120),
dans lequel le protocole d'accès d'urgence est basé sur un calcul multipartite sécurisé (SMC) qui permet une détermination d'une clé d'accès d'urgence (150') sur la base d'une transaction SMC (340) entre le PDDS et au moins un délégué attribué par le patient (110),
dans lequel la clé d'accès d'urgence (150') est une fonction d'un premier secret (352) et d'un second secret (354),
dans lequel le au moins un délégué fournit le premier secret (352) au SMC,
dans lequel le PDDS (100) fournit le second secret (354) au SMC,
dans lequel la transaction SMC (340) ne révèle pas le premier secret (352) au PDDS, et ne révèle pas le second secret (354) à le au moins un délégué,
dans lequel la transaction SMC (340) fournit au moins une sortie de délégué (315) à le au moins un délégué et une sortie de PDDS (325) au PDDS (100),
dans lequel la clé d'accès d'urgence (150') est récupérée sur la base d'une combinaison de la au moins une sortie de délégué (315) et de la sortie PDDS (325),
dans lequel la clé d'accès d'urgence (150') permet le décryptage des informations d'accès cryptées, permettant ainsi au clinicien (120) d'accéder aux données cliniques (130) associées au patient (110).

11. PDDS (100) selon la revendication 10,
dans lequel le au moins un délégué fournit la au moins une sortie de délégué (315) au clinicien (120),
dans lequel le PDDS (100) fournit la sortie de PDDS (325) au clinicien (120),
dans lequel le PDDS (100) fournit les informations d'accès cryptées au clinicien (120),
dans lequel le clinicien récupère la clé d'accès d'urgence (150') en fonction de la au moins une sortie de délégué (315) et de la sortie de PDDS (325) et
dans lequel le clinicien (120) décrypte les informations d'accès cryptées à l'aide de la clé d'accès d'urgence (150').

12. PDDS (100) selon la revendication 10,
dans lequel le au moins un délégué fournit la au moins une sortie de délégué (315) au PDDS,
dans lequel le PDDS (100) récupère la clé d'accès d'urgence (150') sur la base de la combinaison de la au moins une sortie de délégué (315) et de la sortie de PDDS (325), et
dans lequel le PDDS (100) décrypte les informations d'accès cryptées à l'aide de la clé d'accès d'urgence (150') pour produire des informations d'accès décryptées (460), et
dans lequel le PDDS (100) fournit les informations d'accès décryptées (460) au clinicien (120).

13. PDDS (100) selon la revendication 10,
dans lequel le PDDS (100) fournit la sortie de PDDS (325) à le au moins un délégué,
dans lequel le PDDS (100) fournit les informations d'accès cryptées à le au moins un délégué,
dans lequel le au moins un délégué récupère la clé d'accès d'urgence (150') sur la base de la au moins une sortie de délégué (315) et de la sortie de PDDS (325),
dans lequel le au moins un délégué décrypte les informations d'accès cryptées à l'aide de la clé d'accès d'urgence (150'), et
dans lequel le au moins un délégué fournit les informations d'accès décryptées (460) au clinicien (120).

14. PDDS selon la revendication 10, dans lequel le SMC comprend au moins l'un des éléments suivants : un protocole de cryptage homomorphe, un protocole de partage de secret et un protocole de circuit brouillé.

15. Dispositif de récupération de données (DRD) (430) pour récupérer des informations d'accès qui permettent à un clinicien (120) d'accéder à des données cliniques (130) associées à un patient (110) qui sont stockées sur une pluralité de sites médicaux,
dans lequel les informations d'accès sont cryptées par le patient (110) pour fournir des informations d'accès cryptées qui sont stockées dans un système de répertoire de données patient (PDDS) (100),
dans lequel le DRD est actionné par un délégué du patient (110),
dans lequel la clé d'accès d'urgence (150') est une fonction d'un premier secret (352) et d'un second secret (354),
dans lequel au moins une partie du premier secret (352) est stockée sur le DRD,
dans lequel le second secret (354) est stocké sur le PDDS (100),
le DRD comprenant :
un dispositif de commande, dans lequel le dispositif de commande est configuré pour :
recevoir et stocker la au moins une partie du premier secret (352) ; et
exécuter un protocole d'accès d'urgence en coopération avec le PDDS (100) en réponse à une demande du clinicien (120) ;
dans lequel le protocole d'accès d'urgence est basé sur un calcul multipartite sécurisé (SMC) qui permet une détermination de la clé d'accès d'urgence (150') sur la base d'une transaction SMC (340) entre le PDDS (100) et le DRD,
dans lequel la transaction SMC (340) ne révèle pas la au moins une partie du premier secret (352) au PDDS (100), et ne révèle pas le second secret (354) au DRD,
dans lequel la transaction SMC (340) fournit au moins une sortie de délégué (315) au DRD et une sortie de PDDS (325) au PDDS (100),
dans lequel la clé d'accès d'urgence (150') est récupérée sur la base d'une combinaison de la au moins une sortie de délégué (315) et de la sortie de PDDS (325),
dans lequel la clé d'accès d'urgence (150') permet le décryptage des informations d'accès cryptées, permettant ainsi au clinicien (120) d'accéder aux données cliniques (130) associées au patient (110).
